# EUROPEAN PATENT APPLICATION

(11) **EP 2 311 846 A1**
(43) Date of publication of application: **20.04.2011**
(21) Application number: 09794401.1
(22) Date of filing: 06.07.2009
(51) Int. Cl.: C07F 17/02, C07B 53/00, C07C 29/143, C07C 33/46, C07C 201/12, C07C 205/09, C07C 205/16, C07C 247/10, C07C 253/30, C07C 255/12, C07C 255/13, C07C 311/09, C07C 311/18, C07D 307/54, C07D 333/24, C07B 61/00

(54) **CHIRAL IRIDIUM AQUA COMPLEX AND METHOD FOR PRODUCING OPTICALLY ACTIVE HYDROXY COMPOUND USING THE SAME**

(30) Priority: 08.07.2008 JP 2008178040
(71) Applicant: Sumitomo Chemical Company, Limited, Tokyo 104-8260 (JP); Carreira, Erick M., Zurich (CH)
(72) Inventor: CARREIRA, Erick M., Zurich (CH)
(74) Representative: Duckworth, Timothy John
(86) International application number: PCT/JP2009/062289
(87) International publication number: WO 2010/004957

(57) **Abstract**

The present invention provides a novel chiral iridium aqua complex which has good preservation stability, can be easily produced and enables asymmetric transfer hydrogenation in a higher yield and with higher stereoselectivity.

The present invention provides a chiral iridium aqua complex represented by the formula (1A): 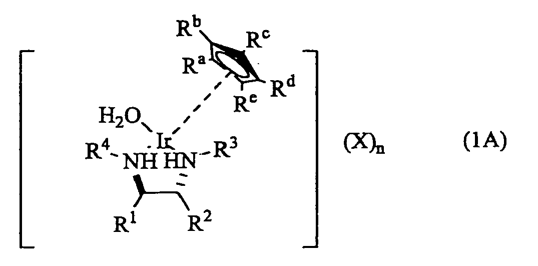 wherein
R^{a}, R^{b}, R^{c}, R^{d} and R^{e} are the same or different and each is a hydrogen atom, a methyl group, an ethyl group or a phenyl group,
R¹ and R² are the same or different and each is an aryl group optionally having substituent(s), or
R¹ and R² in combination show a C₃₋₄ straight chain alkylene group optionally having substituent(s) to form a ring,
R³ is an alkylsulfonyl group optionally having substituent(s) or an arylsulfonyl group optionally having substituent(s),
R⁴ is a hydrogen atom, an alkylsulfonyl group optionally having substituent(s) or an arylsulfonyl group optionally having substituent(s),
X is a monovalent or divalent anion, and
n is 2 when X is a monovalent anion, or n is 1 when X is a divalent anion,
and a production method of an optically active hydroxy compound using the complex.

## Description

### Technical Field

The present invention relates to a novel chiral iridium aqua complex, a production method thereof and a production method of an optically active hydroxy compound or an optically active nitroalkane compound, which comprises asymmetric transfer hydrogenation using the complex.

### Background Art

Conventionally, many methods of producing an optically active hydroxy compound from a carbonyl compound by an asymmetric transfer hydrogenation using a metal complex catalyst have been proposed. For example, non-patent document 1 describes production of an optically active alcohol from an acetophenone derivative by using Ru(II)-TsDPEN (TsDPEN = N-(p-toluenesulfonyl)-1,2-diphenylethylenediamine). Non-patent document 2 describes reduction of ketone by a pH-dependent process using a [Cp^{*}Ir(bpy) (H₂O)]SO₄ (Cp^{*}= h⁵-pentamethylcyclopentadienylanion, bpy =2,2'-bipyridine) complex capable of forming a hydride species in the presence of a formate ion. Recently, a method using a chiral iridium complex has also been proposed. For example, non-patent document 3 describes reduction of ketone by using a complex prepared by reacting CsDPEN (CsDPEN = N-(camphorsulfonyl)-1,2-diphenylethylenediamine) or TsDPEN with [Cp^{*}IrCl₂]₂. Patent document 1 describes reduction of ketone by using a complex prepared by reacting chiral N-substituted sulfonyl-ethylenediamine with [Cp^{*}IrCl₂]₂.

On the other hand, non-patent document 4 describes exchange kinetics and mechanism of ligand in an aqueous solution of a chiral iridium aqua complex. In the document, the complex is exemplified by [Cp^{*}Ir(R,R-DACH) (H₂O)] (ClO₄)₂ (DACH = 1,2-diaminocyclohexane) and [Cp^{*}Ir(R,R-DPEN) (H₂O)] (ClO₄)₂ (DPEN = 1,2-diphenylethane-1,2-diamine).

In addition, patent document 2 and non-patent document 5 propose methods of producing an optically active nitroalkane compound by reducing the double bond of a nitroolefin compound using an organic silicon compound or a thiourea catalyst.

### Citation List

### Patent document

Patent document 1: JP-A-11-335385
Patent document 2: JP-A-2006-524189

### Non-Patent document

Non-Patent document 1: J. Am. Chem. Soc. 1996, 118, 2521-2522
Non-Patent document 2: J. Am. Chem. Soc. 2004, 126, 3020-3021
Non-Patent document 3: Synllet, 2006, 1155-1160
Non-Patent document 4: Eur. J. Inorg. Chem. 2001, 1361-1369
Non-Patent document 5: J. Am. Chem. Soc. 2007, 129, 8976-8977

### Summary of the Invention

### Problems to be Solved by the Invention

The complexes described in the above-mentioned documents have the following problems. Since the complexes of non-patent documents 1 and 3 and patent document 1 are not aqua complexes, and are difficult to dissolve in water depending on the kind of amine, it is difficult to carry out a reaction in an aqueous solvent which is environmentally friendly and suitable for green chemistry desired in recent years. In addition, the ruthenium complex of non-patent document 1 shows problematically slow progress of the reaction as compared to an iridium catalyst. The complex of non-patent document 2 is associated with a problem of unapplicability to an asymmetric reaction since the ligand is not chiral. The complex of non-patent document 4 may explode since it is perchlorate, and it requires close attention in handling. Moreover, non-patent document 4 does not teach at all reaction for which the complex is to be used. An asymmetric transfer hydrogenation is generally carried out in the presence of formic acid or a salt thereof as a hydrogen source. However, contact of the complex with perchlorate is presumed to be dangerous, and perchlorate cannot be applied to such a reaction.

As described above, a chiral iridium aqua complex suitable for an asymmetric transfer hydrogenation has not been proposed up to the present.

The present invention provides a chiral iridium aqua complex that can be produced easily and enables safe asymmetric transfer hydrogenation in an aqueous solvent.

### Means of Solving the Problems

The above-mentioned problems were intensively studied and, as a result, it has been found that chiral iridium aqua complexes of the following formulas (1A) and (1) can be produced easily, an asymmetric transfer hydrogenation using the complex can be carried out safely in an aqueous solvent, and the complexes have good preservation stability and achieve higher yield and higher stereoselectivity in an asymmetric transfer hydrogenation as compared to conventional iridium complexes, which resulted in the completion of the invention. Accordingly, the present invention provides the following.

[1] A chiral iridium aqua complex represented by the formula (1A):

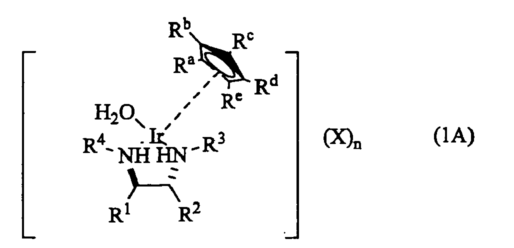

wherein
R^{a}, R^{b}, R^{c}, R^{d} and R^{e} are the same or different and each is a hydrogen atom, a methyl group, an ethyl group or a phenyl group,
R¹ and R² are the same or different and each is an aryl group optionally having substituent(s), or
R¹ and R² in combination show a C₃₋₄ straight chain alkylene group optionally having substituent(s) to form a ring,
R³ is an alkylsulfonyl group optionally having substituent(s) or an arylsulfonyl group optionally having substituent(s),
R⁴ is a hydrogen atom, an alkylsulfonyl group optionally having substituent(s) or an arylsulfonyl group optionally having substituent(s),
X is a monovalent or divalent anion, and
n is 2 when X is a monovalent anion, or n is 1 when X is a divalent anion,
(hereinafter to be referred to as chiral iridium aqua complex (1A)).
[2] A chiral iridium aqua complex represented by the formula (1):

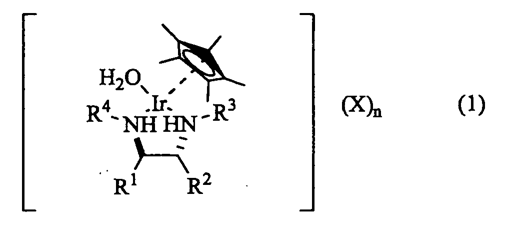

wherein
R¹ and R² are the same or different and each is an aryl group optionally having substituent(s), or
R¹ and R² in combination show a C₃₋₄ straight chain alkylene group optionally having substituent(s) to form a ring,
R³ is an alkylsulfonyl group optionally having substituent(s) or an arylsulfonyl group optionally having substituent(s),
R⁴ is a hydrogen atom, an alkylsulfonyl group optionally having substituent(s) or an arylsulfonyl group optionally having substituent(s),
X is a monovalent or divalent anion, and
n is 2 when X is a monovalent anion, or n is 1 when X is a divalent anion,
(hereinafter to be referred to as chiral iridium aqua complex (1)).
[3] The chiral iridium aqua complex of the above-mentioned [1] or [2], wherein
   R¹ and R² are the same or different and each is a C₆₋₁₀ aryl group optionally having'substituent(s) selected from a halogen atom, a C₁₋₆ alkyl group, a C₁₋₆ haloalkyl group, a C₁₋₆ alkoxy group and a C₁₋₆ haloalkoxy group; or
   R¹ and R² in combination show a C₃₋₄ straight chain alkylene group optionally having substituent(s) to form a ring.
[4] The chiral iridium aqua complex of the above-mentioned [1] or [2], wherein R¹ and R² are the same or different and each is a C₆₋₁₀ aryl group optionally having substituent(s) selected from a halogen atom, a C₁₋₆ alkyl group, a C₁₋₆ haloalkyl group, a C₁₋₆ alkoxy group and a C₁₋₆ haloalkoxy group.
[5] The chiral iridium aqua complex of the above-mentioned [1] or [2], wherein R¹ and R² are the same or different and each is phenyl optionally having fluorine atom(s).
[6] The chiral iridium aqua complex of the above-mentioned [1] or [2], wherein R³ is a C₆₋₁₀) arylsulfonyl group optionally having substituent(s) selected from a halogen atom, a C₁₋₆ alkyl group, a C₁₋₆ haloalkyl group and a nitro group; or a C₁₋₆ alkylsulfonyl group optionally having halogen atom(s).
[7] The chiral iridium aqua complex of the above-mentioned [1] or [2], wherein R³ is phenylsulfonyl having substituent(s) selected from a fluorine atom, trifluoromethyl and nitro; or a C₁₋₄ alkylsulfonyl group having fluorine atom(s).
[8] The chiral iridium aqua complex of the above-mentioned [1] or [2], wherein R⁴ is a hydrogen atom; or a C₆₋₁₀ arylsulfonyl group optionally having C₁₋₆ alkyl group (s) .
[9] The chiral iridium aqua complex of the above-mentioned [1] or [2], wherein R⁴ is a hydrogen atom.
[10] The chiral iridium aqua complex of the above-mentioned [1] or [2], wherein X is a sulfate ion.
[11] A chiral iridium aqua complex represented by formula:

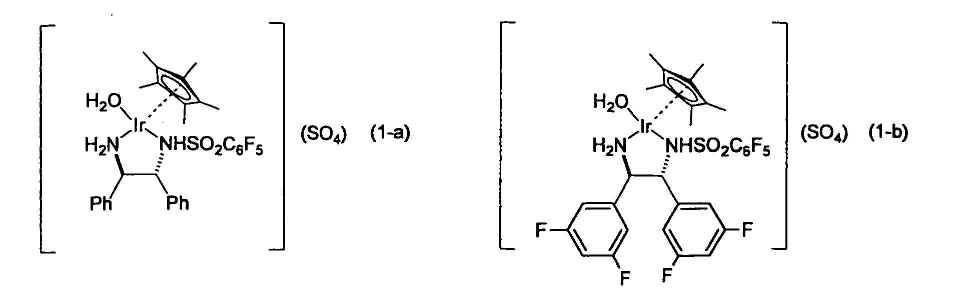

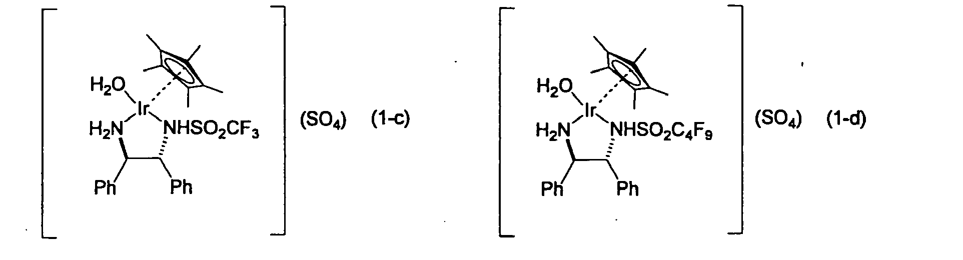

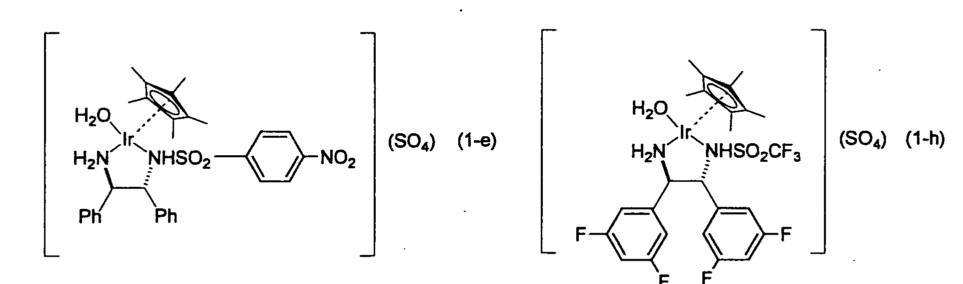

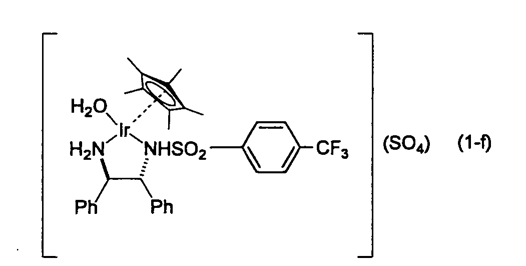

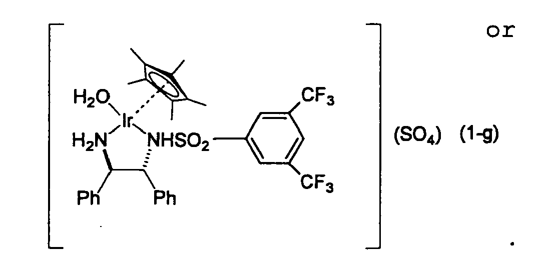

[12] A chiral iridium aqua complex represented by formula:

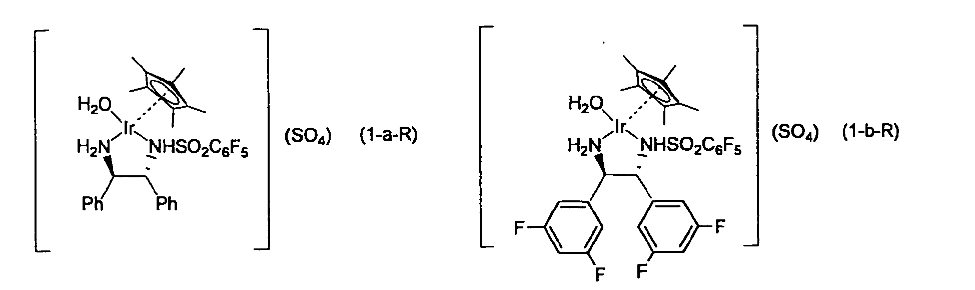

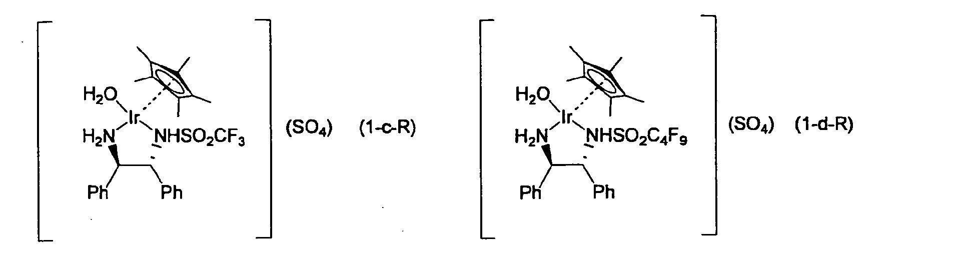

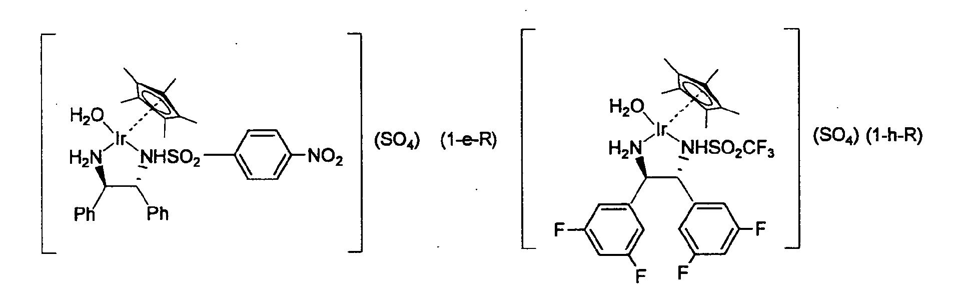

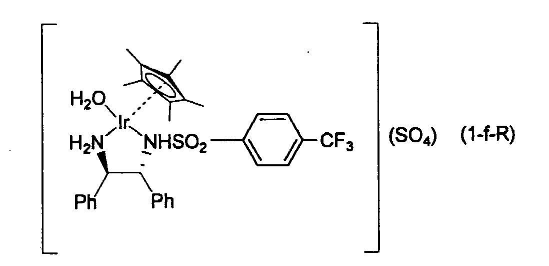

or

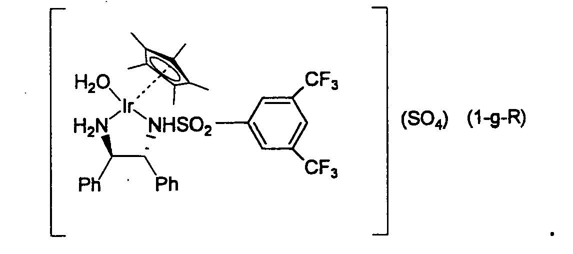

[13] A chiral iridium aqua complex represented by formula:

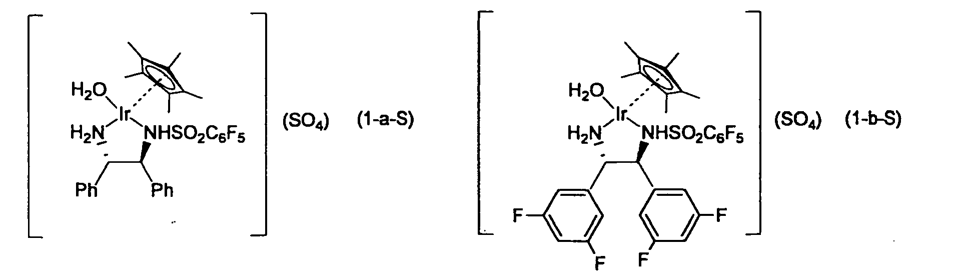

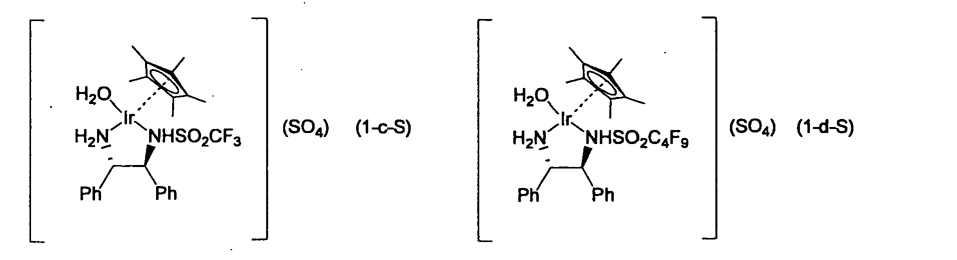

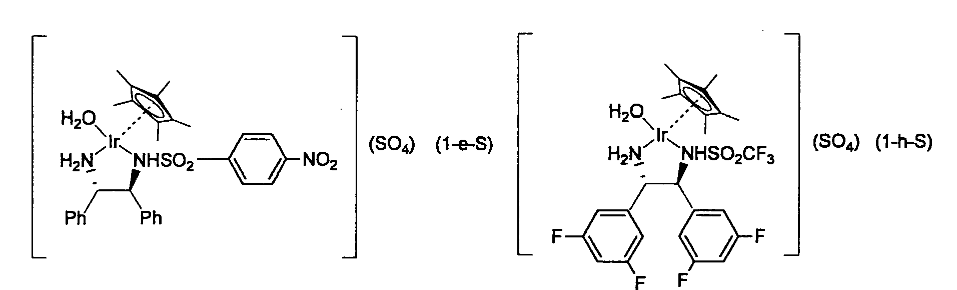

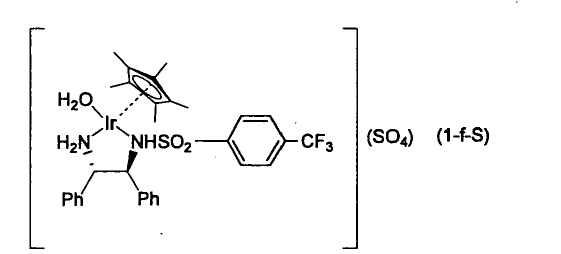

or

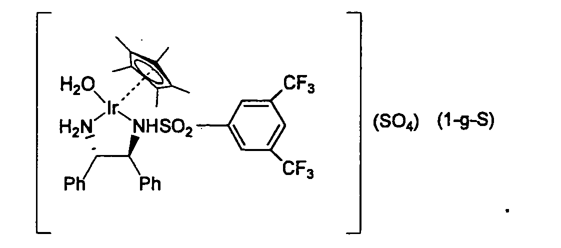

[14] A chiral iridium aqua complex represented by formula:

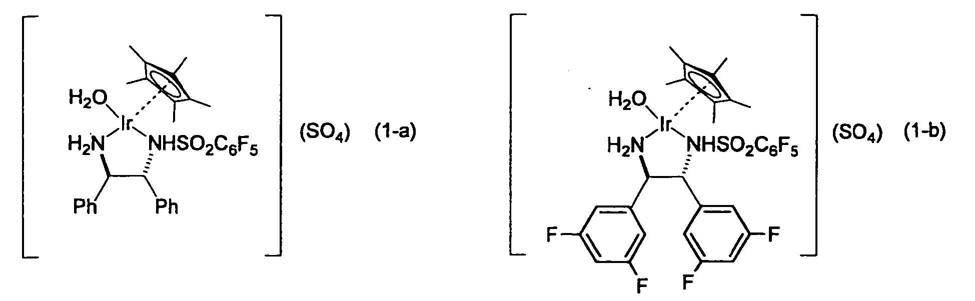

or

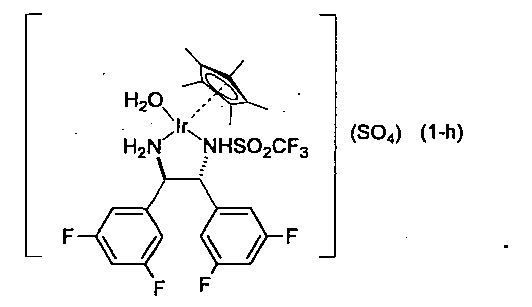

[15] A chiral iridium aqua complex represented by formula:

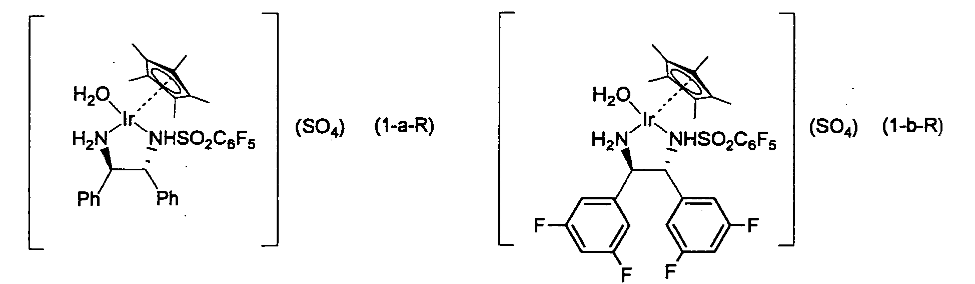

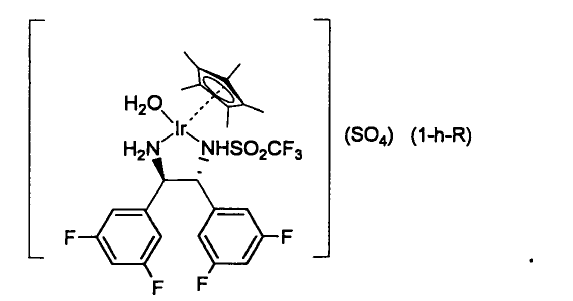

or

[16] A chiral iridium aqua complex represented by formula:

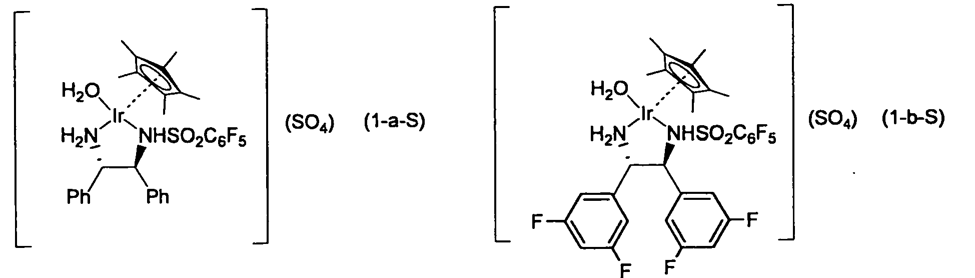

or

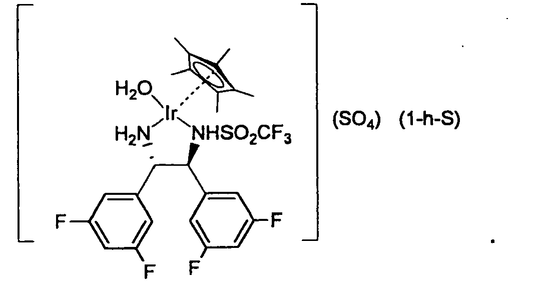

[17] A method of producing a chiral iridium aqua complex represented by the formula (1A), which comprises reacting an iridium complex represented by the formula (2) (hereinafter to be referred to as iridium complex (2)) with a chiral diamine represented by the formula (3) (hereinafter to be referred to as chiral diamine (3)):

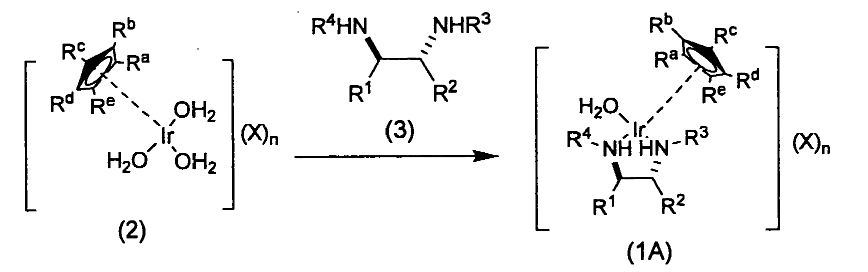

wherein
R^{a}, R^{b}, R^{c}, R^{d} and R^{e} are the same or different and each is a hydrogen atom, a methyl group, an ethyl group or a phenyl group,
R¹ and R² are the same or different and each is an aryl group optionally having substituent(s), or
R¹ and R² in combination show a C₃₋₄ straight chain alkylene group optionally having substituent(s) to form a ring,
R³ is an alkylsulfonyl group optionally having substituent(s) or an arylsulfonyl group optionally having substituent(s),
R⁴ is a hydrogen atom, an alkylsulfonyl group optionally having substituent(s) or an arylsulfonyl group optionally having substituent(s),
X is a monovalent or divalent anion, and
n is 2 when X is a monovalent anion, or n is 1 when X is a divalent anion.

[18] A method of producing an optically active hydroxy compound represented by the formula (5) (hereinafter to be referred to as optically active hydroxy compound (5)), which comprises subjecting a carbonyl compound represented by the formula (4) (hereinafter to be referred to as carbonyl compound (4)) to an asymmetric transfer hydrogenation in the presence of the chiral iridium aqua complex of any of the above-mentioned [1] to [16]:

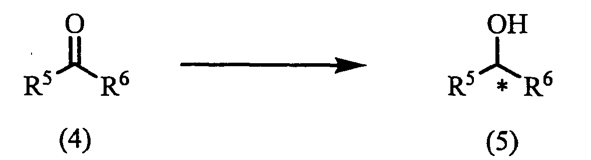

wherein
R⁵ is an aryl group optionally having substituent(s), a heteroaryl group optionally having substituent(s), a cycloalkyl group optionally having substituent(s) or an aralkyl group optionally having substituent(s),
R⁶ is a carboxyl group, a carbamoyl group optionally having substituent(s) or an alkyl group optionally having substituent(s), and
the carbon atom marked with * is an asymmetric carbon atom.
[19] The method of the above-mentioned [18], wherein the chiral iridium aqua complex is

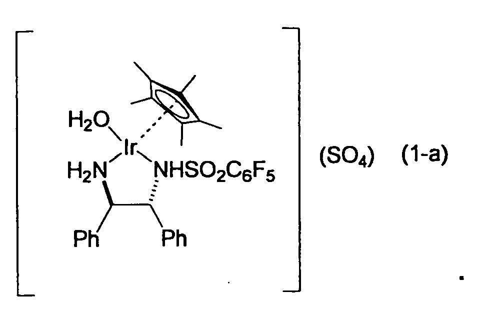

[20] The method of the above-mentioned [18], wherein the chiral iridium aqua complex is

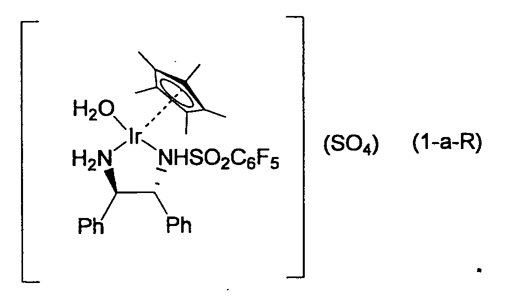

[21] The method of the above-mentioned [18], wherein the chiral iridium aqua complex is

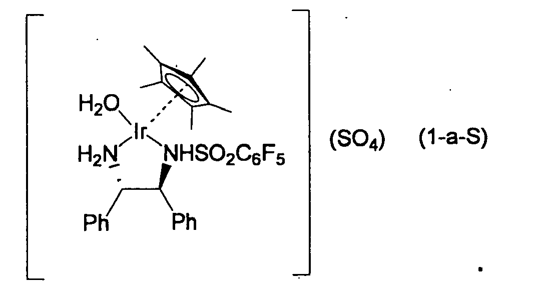

[22] The method of the above-mentioned [18], wherein R⁵ is a C₆₋₁₀ aryl group optionally having substituent(s) selected from a halogen atom, a C₁₋₆ alkyl group, a C₁₋₆ alkoxy group, a cyano group and nitro group; a C₃₋₈ cycloalkyl group; or a 5- or 6-membered heteroaryl group optionally having C₁₋₆ alkyl group(s).
[23] The method of the above-mentioned [18], wherein R⁶ is a C₁₋₆ alkyl group optionally having substituent(s) selected from a halogen atom, a cyano group, a nitro group and an azido group.
[24] The method of the above-mentioned [18], wherein the asymmetric transfer hydrogenation is carried out in the presence of formic acid or a salt thereof.
[25] The method of the above-mentioned [18], wherein the asymmetric transfer hydrogenation is carried out in the presence of formic acid.
[26] The method of the above-mentioned [18], wherein the asymmetric transfer hydrogenation is carried out under the condition of pH 2 to 5.

[27] A method of producing an optically active nitroalkane compound represented by the formula (7) (hereinafter to be referred to as optically active nitroalkane compound (7)), which comprises subjecting a nitroolefin compound represented by the formula (6) (hereinafter to be referred to as nitroolefin compound (6)) to an asymmetric transfer hydrogenation in the presence of the chiral iridium aqua complex of any of the above-mentioned [1] to [16]:

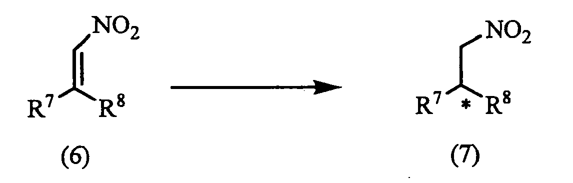

wherein
R⁷ is an aryl group optionally having substituent(s) or a heteroaryl group optionally having substituent(s),
R⁸ is an alkyl group optionally having substituent(s), an aryl group optionally having substituent(s) or a heteroaryl group optionally having substituent(s), and
the carbon atom marked with * is an asymmetric carbon atom.
[28] The method of the above-mentioned [27], wherein the chiral iridium aqua complex is

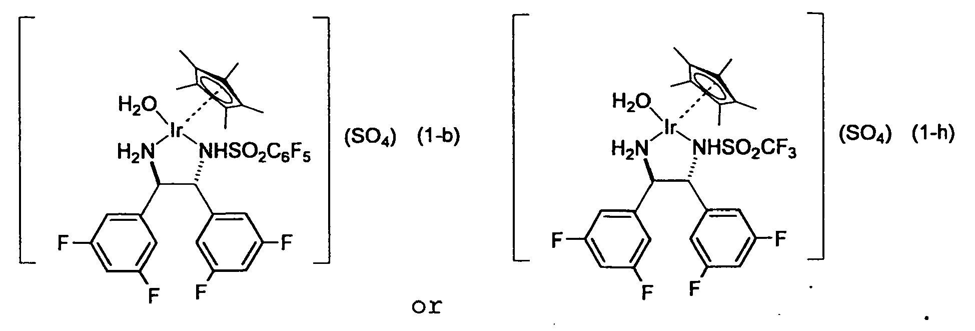

[29] The method of the above-mentioned [27], wherein the chiral iridium aqua complex is

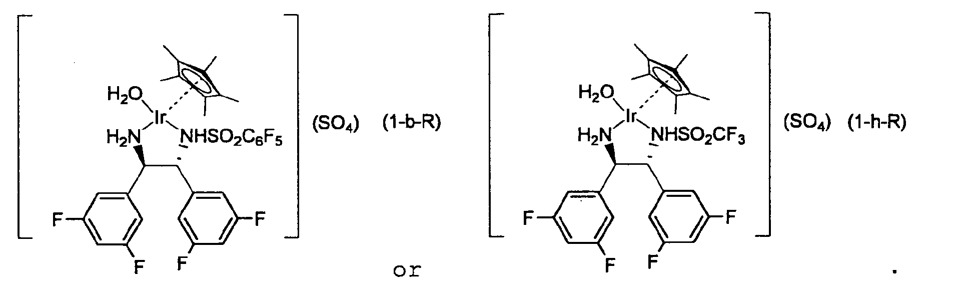

[30] The method of the above-mentioned [27], wherein the chiral iridium aqua complex is

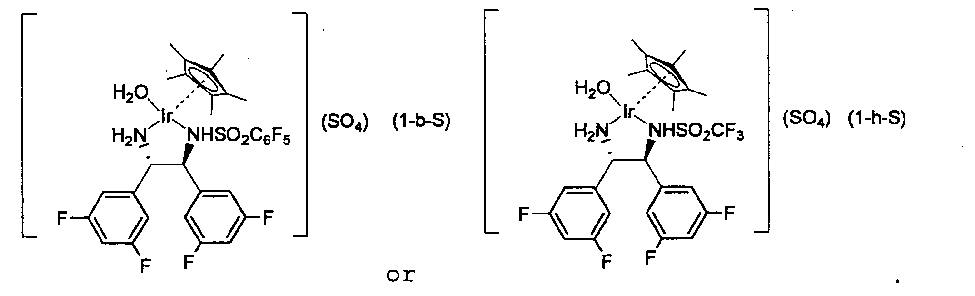

[31] The method of the above-mentioned [27], wherein R⁷ is a C₆₋₁₀ aryl group optionally having substituent(s) selected from a halogen atom, a C₁₋₆ alkyl group, a C₁₋₆ haloalkyl group and a C₁₋₆ alkoxy group.
[32] The method of the above-mentioned [27], wherein R⁸ is a C₁₋₆ alkyl group.
[33] The method of the above-mentioned [27], wherein the asymmetric transfer hydrogenation is carried out in the presence of formic acid or a salt thereof.
[34] The method of the above-mentioned [27], wherein the asymmetric transfer hydrogenation is carried out in the presence of formic acid.
[35] The method of the above-mentioned [27], wherein the asymmetric transfer hydrogenation is carried out under the condition of pH 2 to 5.

[36] A chiral diamine represented by formula:

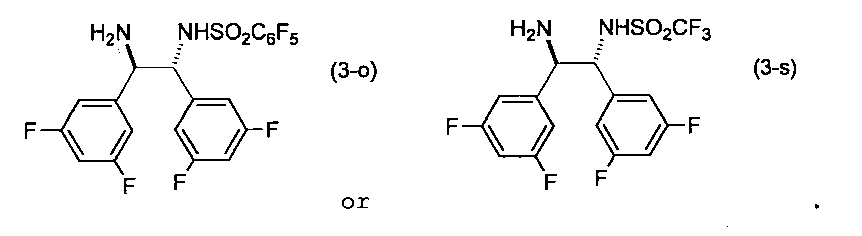

[37] A chiral diamine represented by formula:

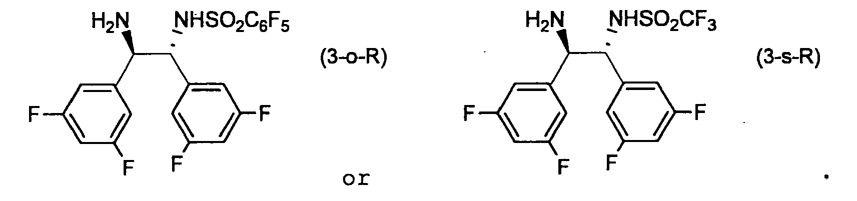

[38] A chiral diamine represented by formula:

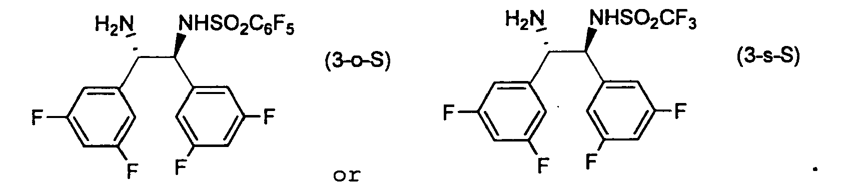

### Effect of the Invention

Chiral iridium aqua complex (1A) of the present invention can be produced easily. In addition, since the complex has good solubility in water, the asymmetric transfer hydrogenation can be carried out in an aqueous solvent which is environmentally friendly and suitable for green chemistry. Even if the asymmetric transfer hydrogenation is carried out in the presence of formic acid or a salt thereof, it can be performed safely since the danger caused by hydrogen generation and the like is absent.
Moreover, the chiral iridium aqua complex (1A) of the present invention has good stability to the air and water. Moreover, the asymmetric transfer hydrogenation using the chiral iridium aqua complex (1A) of the present invention can achieve higher stereoselectivity and higher yield, as compared to conventional iridium complexes.

### Description of Embodiments

The present invention is explained in detail in the following.
R^{a}, R^{b}, R^{c}, R^{d} and R^{e} are preferably the same or different and each is a hydrogen atom or a methyl group. More preferably, three to five of R^{a}, R^{b}, R^{c}, R^{d} and R^{e} are methyl groups, and the remaining group(s) is/are hydrogen atom(s). Particularly preferably, all of R^{a}, R^{b}, R^{c}, R^{d} and R^{e} are methyl groups (i.e., chiral iridium aqua complex (1)) .

The "aryl group" of the "aryl group optionally having substituent(s)" for R¹ or R² is preferably a C₆₋₁₄ aryl group, and examples thereof include phenyl, naphthyl, anthryl, phenanthryl, acenaphthyl, biphenylyl and the like. Among them, a C₆₋₁₀ aryl group is preferable, phenyl and naphthyl are more preferable, and phenyl is particularly preferable.
Examples of the "substituent" of the "aryl group optionally having substituent(s)" include a halogen atom, a C₁₋₆ alkyl group, a C₁₋₆ haloalkyl group, a C₁₋₆ alkoxy group, a C₁₋₆ haloalkoxy group, a nitro group, a carboxyl group, a cyano group, an azido group and the like.

Examples of the halogen atom include fluorine, chlorine, bromine and iodine.
The C₁₋₆ alkyl group may be straight chain or branched chain, and examples thereof include methyl, ethyl, propyl, isopropyl, butyl, isobutyl, sec-butyl, tert-butyl, pentyl, isopentyl, neopentyl, hexyl and the like.
The C₁₋₆ haloalkyl group may be straight chain or branched chain, and examples thereof include fluoromethyl, difluoromethyl, trifluoromethyl, 2-fluoroethyl, 2,2-difluoroethyl, 2,2,2-trifluoroethyl, 3-fluoropropyl, 4-fluorobutyl, 5-fluoropentyl, 6-fluorohexyl and the like.
The C₁₋₆ alkoxy group may be straight chain or branched chain, and examples thereof include methoxy, ethoxy, propoxy, isopropoxy, butoxy, isobutoxy, sec-butoxy, tert-butoxy, pentyloxy, isopentyloxy, neopentyloxy, hexyloxy and the like.
The C₁₋₆ haloalkoxy group may be straight chain or branched chain, and examples thereof include fluoromethoxy, difluoromethoxy, trifluoromethoxy, 2-fluoroethoxy, 2,2-difluoroethoxy, 2,2,2-trifluoroethoxy, 3-fluoropropoxy, 4-fluorobutoxy, 5-fluoropentyloxy, 6-fluorohexyloxy and the like.

As the "substituent" of the "aryl group optionally having substituent(s)" for R¹ or R², a halogen atom, a C₁₋₆ alkyl group, a C₁₋₆ haloalkyl group, a C₁₋₆ alkoxy group and a C₁₋₆ haloalkoxy group are preferable, a fluorine atom, methyl, trifluoromethyl, methoxy and trifluoromethoxy are more preferable, and a fluorine atom is particularly preferable. When two or more substituents are present, these substituents may be the same or different.

Examples of the "C₃₋₄ straight chain alkylene group" of the "C₃₋₄ straight chain alkylene group optionally having substituent(s)" such that R¹ and R² in combination form a ring include trimethylene and tetramethylene. Examples of the ring formed by the C₃₋₄ straight chain alkylene group include cyclopentane and cyclohexane. Examples of the "substituent" of the "C₃₋₄ straight chain alkylene group optionally having substituent(s)" include the same group as the above-mentioned "substituent" of the "aryl group optionally having substituent(s)". When two or more substituents are present, these substituents may be the same or different.

R¹ and R² are preferably the same or different and each is a C₆₋₁₀ aryl group optionally having substituent(s) selected from a halogen atom, a C₁₋₆ alkyl group, a C₁₋₆ haloalkyl group, a C₁₋₆ alkoxy group and a C₁₋₆ haloalkoxy group; or R¹ and R² in combination show a C₃₋₄ straight chain alkylene group optionally having substituent(s) to form a ring,
more preferably a C₆₋₁₀ aryl group optionally having substituent(s) selected from a halogen atom, a C₁₋₆ alkyl group, a C₁₋₆ haloalkyl group, a C₁₋₆ alkoxy group and a C₁₋₆ haloalkoxy group,
further more preferably phenyl or naphthyl, each of which optionally has substituent(s) selected from a fluorine atom, methyl, trifluoromethyl, methoxy and trifluoromethoxy,
still more preferably phenyl optionally having substituent(s) selected from a fluorine atom, methyl, trifluoromethyl, methoxy and trifluoromethoxy,
particularly preferably phenyl optionally having fluorine atom(s).
Most preferably, both of R¹ and R² are unsubstituted phenyl or 3,5-difluorophenyl.

Examples of the "alkylsulfonyl group" of the "alkylsulfonyl group optionally having substituent(s)" for R³ or R⁴ include a straight chain or branched chain C₁₋₁₀ alkylsulfonyl group such as methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, hexylsulfonyl, 2-ethylbutylsulfonyl, heptylsulfonyl, octylsulfonyl, nonylsulfonyl, decylsulfonyl and the like. Among them, a C₁₋₆ alkylsulfonyl group is preferable, and a C₁₋₄ alkylsulfonyl group is particularly preferable.

Examples of the "substituent" of the "alkylsulfonyl group optionally having substituent(s)" include a halogen atom, a C₁₋₆ haloalkyl group, a C₁₋₆ alkoxy group, a C₁₋₆ haloalkoxy group, a nitro group, a carboxyl group, a cyano group, an azido group and the like. When two or more substituents are present, these substituents may be the same or different.

As the "substituent" of the "alkylsulfonyl group optionally having substituent(s)" for R³ or R⁴, a halogen atom is preferable, and a fluorine atom is particularly preferable.

Examples of the aryl moiety of the "arylsulfonyl group" of the "arylsulfonyl group optionally having substituent(s)" for R³ or R⁴ include the same group as the "aryl group" of the "aryl group optionally having substituent(s)" for R¹ or R². Specific examples of the "arylsulfonyl group" include phenylsulfonyl, naphthylsulfonyl, anthrylsulfonyl, phenanthrylsulfonyl, acenaphthylsulfonyl, biphenylylsulfonyl and the like. Among them, a C₆₋₁₀ arylsulfonyl group is preferable, phenylsulfonyl and naphthylsulfonyl are more preferably, and phenylsulfonyl is particularly preferable. Examples of the "substituent" of the "arylsulfonyl group optionally having substituent(s)" include the same group as the "substituent" of the "aryl group optionally having substituent(s)" for R¹ or R². When two or more substituents are present, these substituents may be the same or different.

As the "substituent" of the "arylsulfonyl group optionally having substituent(s)" for R³ or R⁴, a halogen atom, a C₁₋₆ alkyl group, a C₁₋₆ haloalkyl group and a nitro group are preferable, a fluorine atom, a chlorine atom, a C₁₋₄ alkyl group, trifluoromethyl and nitro are more preferable, a fluorine atom, trifluoromethyl and nitro are further more preferable, and a fluorine atom is particularly preferable.

R³ is preferably a C₆₋₁₀ arylsulfonyl group optionally having substituent(s) selected from a halogen atom, a C₁₋₆ alkyl group, a C₁₋₆ haloalkyl group and a nitro group; or a C₁₋₆ alkylsulfonyl group optionally having halogen atom(s),
more preferably phenylsulfonyl or naphthylsulfonyl, each of which optionally has substituent(s) selected from a fluorine atom, a chlorine atom, a C₁₋₄ alkyl group, trifluoromethyl and nitro; or a C₁₋₄ alkylsulfonyl group optionally having fluorine atom(s),
further more preferably phenylsulfonyl having substituent(s) selected from a fluorine atom, trifluoromethyl and nitro; or a C₁₋₄ alkylsulfonyl group having fluorine atom(s), still more preferably pentafluorophenylsulfonyl, 4-(trifluoromethyl)phenylsulfonyl, 3,5-bis(trifluoromethyl)phenylsulfonyl, 4-nitrophenylsulfonyl; or a perfluoro-substituted C₁₋₄ alkylsulfonyl group,
particularly preferably pentafluorophenylsulfonyl.

R⁴. is preferably a hydrogen atom; or a C₆₋₁₀ arylsulfonyl group optionally having C₁₋₆ alkyl group(s),
more preferably a hydrogen atom; or phenylsulfonyl optionally having methyl,
further more preferably a hydrogen atom; or p-toluenesulfonyl, particularly preferably a hydrogen atom.

Examples of the "aryl group optionally having substituent(s)" for R⁵ include the same group as the "aryl group optionally having substituent(s)" for R¹ or R². When two or more substituents are present, these substituents may be the same or different.

The "aryl group" of the "aryl group optionally having substituent(s)" for R⁵ is preferably phenyl or naphthyl. As the "substituent" of the "aryl group optionally having substituent(s)", a halogen atom, a C₁₋₆ alkyl group, a C₁₋₆ alkoxy group, a cyano group and a nitro group are preferable, and a halogen atom, a C₁₋₄ alkyl group, a C₁₋₄ alkoxy group, a cyano group and a nitro group are more preferable.

Examples of the "heteroaryl group" of the "heteroaryl group optionally having substituent(s)" for R⁵ include furyl, thienyl, pyrrolyl, pyridyl, benzofuranyl, indolyl, benzothiophenyl, pyrimidyl, pyrazinyl, quinolyl, isoquinolyl, phthalazinyl, quinazolinyl, quinoxalinyl, cinnolinyl and the like. Among them, furyl and thienyl are preferable. Examples of the "substituent" of the "heteroaryl group optionally having substituent(s)" include the same group as the "substituent" of the "aryl group optionally having substituent(s)" for R¹ or R². When two or more substituents are present, these substituents may be the same or different.

As the "substituent" of the "heteroaryl group optionally having substituent(s)", a C₁₋₆ alkyl group is preferable, and a C₁₋₄ alkyl group is more preferable.

Examples of the "cycloalkyl group" of the "cycloalkyl group optionally having substituent(s)" for R⁵ include a C₃₋₈ cycloalkyl group such as cyclopropyl, cyclobutyl, cyclopentyl, cyclohexyl, cycloheptyl, cyclooctyl and the like. Among them, a C₅₋₆ cycloalkyl group is preferable, and cyclohexyl is particularly preferable. Examples of the "substituent" of the "cycloalkyl group optionally having substituent(s) include the same group as the "substituent" of the "aryl group optionally having substituent(s)" for R¹ or R². When two or more substituents are present, these substituents may be the same or different.

Examples of the aryl moiety of the "aralkyl group" of the "aralkyl group optionally having substituent(s)" for R⁵ include the same group as the "aryl group" of the "aryl group optionally having substituent(s)" for R¹ or R², and examples of the alkyl moiety include a C₁₋₁₀ straight chain or branched chain alkyl group such as methyl, ethyl, propyl, isopropyl, butyl, isobutyl, sec-butyl, tert-butyl, pentyl, isopentyl, neopentyl, hexyl, 2-ethylbutyl, heptyl, octyl, nonyl, decyl and the like. Specific examples of the "aralkyl group" include benzyl, 1-phenylethyl, 2-phenylethyl, 1-(1-naphthyl)ethyl, 1-(2-naphthyl)ethyl, 2-(1-naphthyl)ethyl, 2-(2-naphthyl)ethyl, 1-phenylpropyl, 2-phenylpropyl, 3-phenylpropyl, 1-phenylbutyl, 2-phenylbutyl, 3-phenylbutyl, 4-phenylbutyl and the like. Examples of the "substituent" of the "aralkyl group optionally having substituent(s)" include the same group as the "substituent" of the "aryl group optionally having substituent(s)" for R¹ or R². When two or more substituents are present, these substituents may be the same or different.

R⁵ is preferably an aryl group optionally having substituent(s), a heteroaryl group optionally having substituent(s) or a cycloalkyl group optionally having substituent(s), more preferably an aryl group optionally having substituent(s), a heteroaryl group optionally having substituent(s) or a cycloalkyl group,
further more preferably a C₆₋₁₀ aryl group optionally having substituent(s) selected from a halogen atom, a C₁₋₆ alkyl group, a C₁₋₆ alkoxy group, a cyano group and a nitro group; a C₃₋₈ cycloalkyl group; or a 5- or 6-membered heteroaryl group optionally having C₁₋₆ alkyl group(s),
further more preferably a C₆₋₁₀ aryl group optionally having substituent(s) selected from a halogen atom, a C₁₋₄ alkyl group, a C₁₋₄ alkoxy group, a cyano group and a nitro group; a C₅₋₆ cycloalkyl group; or a 5- or 6-membered heteroaryl group optionally having C₁₋₄ alkyl group(s),
still more preferably a C₆₋₁₀ aryl group optionally having substituent(s) selected from a halogen atom, a C₁₋₄ alkyl group, a C₁₋₄ alkoxy group, a cyano group and a nitro group; or a 5- or 6-membered heteroaryl group optionally having C₁₋₄ alkyl group(s),
particularly preferably a C₆₋₁₀ aryl group optionally having substituent(s) selected from a halogen atom, a C₁₋₄ alkyl group, a C₁₋₄ alkoxy group, a cyano group and a nitro group.

Examples of the "substituent" of the "carbamoyl group optionally having substituent(s)" for R⁶ include a halogen atom, a C₁₋₆ alkyl group, a C₁₋₆ alkoxy group and the like.

Examples of the "alkyl group" of the "alkyl group optionally having substituent(s)" for R⁶ include a C₁₋₁₀ straight chain or branched chain alkyl group such as methyl, ethyl, propyl, isopropyl, butyl, isobutyl, sec-butyl, tert-butyl, pentyl, isopentyl, neopentyl, hexyl, 2-ethylbutyl, heptyl, octyl, nonyl, decyl and the like. Among them, a C₁₋₆ alkyl group is preferable, a C₁₋₄ alkyl group is more preferable, and methyl is particularly preferable. Examples of the "substituent" of the "alkyl group optionally having substituent(s)" include a halogen atom, a C₁₋₆ haloalkyl group, a C₁₋₆ alkoxy group, a C₁₋₆ haloalkoxy group, a nitro group, a carboxyl group, a cyano group, an azido group and the like. Among them, a halogen atom, a cyano group, a nitro group and an azido group are preferable.

R⁶ is preferably a C₁₋₆ alkyl group optionally having substituent(s) selected from a halogen atom, a cyano group, a nitro group and an azido group, more preferably a C₁₋₄ alkyl group optionally having substituent(s) selected from a halogen atom, a cyano group, a nitro group and an azido group.

Examples of the "aryl group optionally having substituent(s)" for R⁷ include the same group as the "aryl group optionally having substituent(s)" for R¹ or R². When two or more substituents are present, these substituents may be the same or different.

The "aryl group" of the "aryl group optionally having substituent(s)" for R⁷ is preferably phenyl or naphthyl. As the "substituent" of the "aryl group optionally having substituent(s)", a halogen atom, a C₁₋₆ alkyl group, a C₁₋₆ haloalkyl group and a C₁₋₆ alkoxy group are preferable, and a halogen atom, a C₁₋₄ alkyl group, a C₁₋₄ haloalkyl group and a C₁₋₄ alkoxy group are more preferable.

Examples of the "heteroaryl group optionally having substituent(s)" for R⁷ include the same group as the "heteroaryl group optionally having substituent(s)" for R⁵. When two or more substituents are present, these substituents may be the same or different.

R⁷ is preferably a C₆₋₁₀ aryl group optionally having substituent(s) selected from a halogen atom, a C₁₋₆ alkyl group, a C₁₋₆ haloalkyl group and a C₁₋₆ alkoxy group, more preferably phenyl or naphthyl, each of which optionally has substituent(s) selected from a halogen atom, a C₁₋₄ alkyl group, a C₁₋₄ haloalkyl group and a C₁₋₄ alkoxy group.

Examples of the "alkyl group optionally having substituent(s)" for R⁸ include the same group as the "alkyl group optionally having substituent(s)" for R⁶. When two or more substituents are present, these substituents may be the same or different.

The "alkyl group" of the "alkyl group optionally having substituent(s)" for R⁸ is preferably a C₁₋₆ alkyl group, more preferably a C₁₋₄ alkyl group, particularly preferably methyl.

Examples of the "aryl group optionally having substituent(s)" for R⁸ include the same group as the "aryl group optionally having substituent(s)" for R¹ or R². When two or more substituents are present, these substituents may be the same or different.

Examples of the "heteroaryl group optionally having substituent(s)" for R⁸ include the same group as the "heteroaryl group optionally having substituent(s)" for R⁷. When two or more substituents are present, these substituents may be the same or different.

R⁸ is preferably a C₁₋₆ alkyl group, more preferably a C₁₋₄ alkyl group, particularly preferably methyl.

Examples of the anion for X include a chloride ion, a bromide ion, an iodide ion, a methanesulfonate ion, a trifluoromethanesulfonate ion, a formate ion, an acetate ion, a trichloroacetate ion, a nitrate ion, a sulfate ion, an acetylacetonate ion, a hexafluorophosphate ion, a tetrafluoroborate ion and the like. Among them, a sulfate ion is preferable.

* means that the carbon atom marked therewith is an asymmetric carbon atom, and the ratio of a compound wherein the configuration of the substituents on the asymmetric carbon atom is R-configuration and a compound wherein the configuration of the substituents bonded to the asymmetric carbon atom is S-configuration is any of 0:100 to 100:0 (excluding 50:50).

Among chiral iridium aqua complex (1A) of the present invention, chiral iridium aqua complex (1) is preferable, and preferable specific examples thereof include the following chiral iridium aqua complexes (1-a) to (1-h):

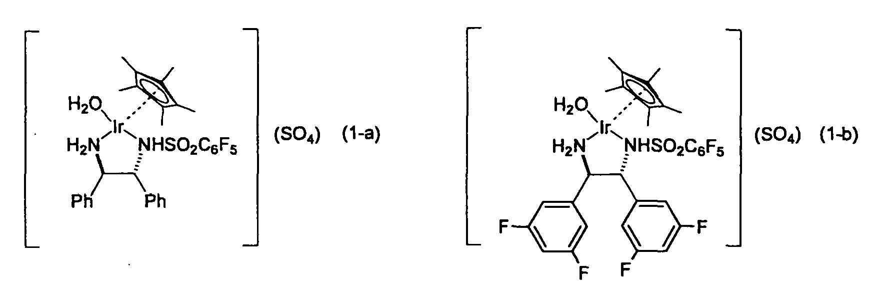

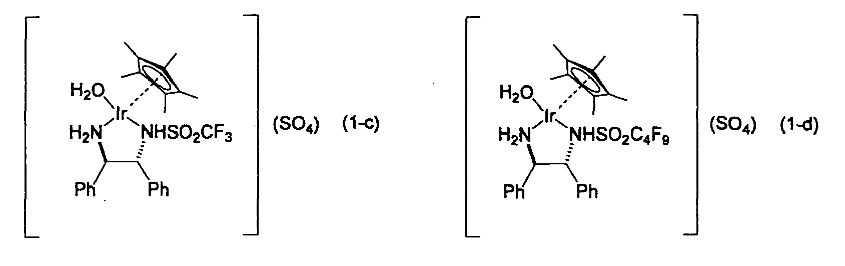

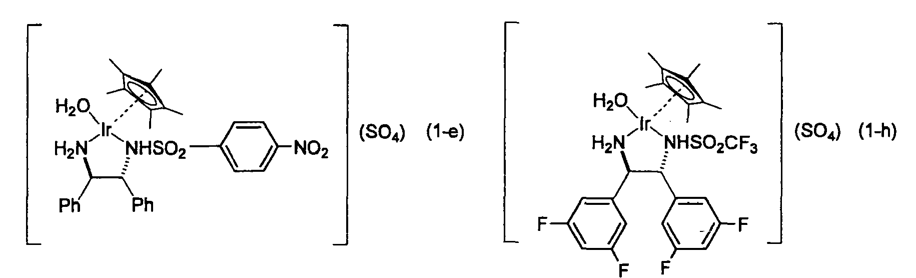

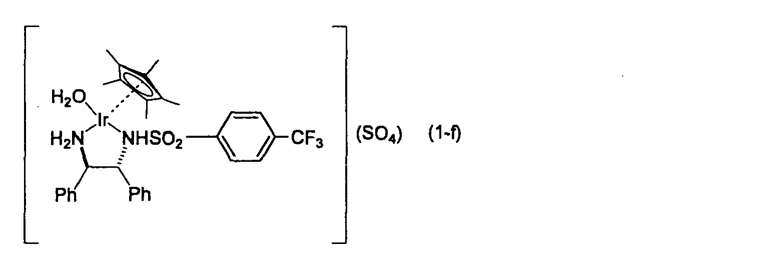

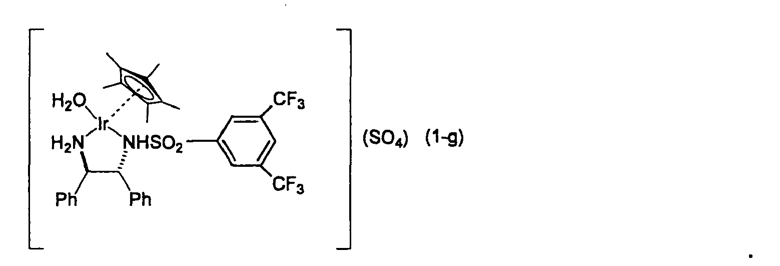

That is, the following chiral iridium aqua complexes (1-a-R) to (1-h-R):

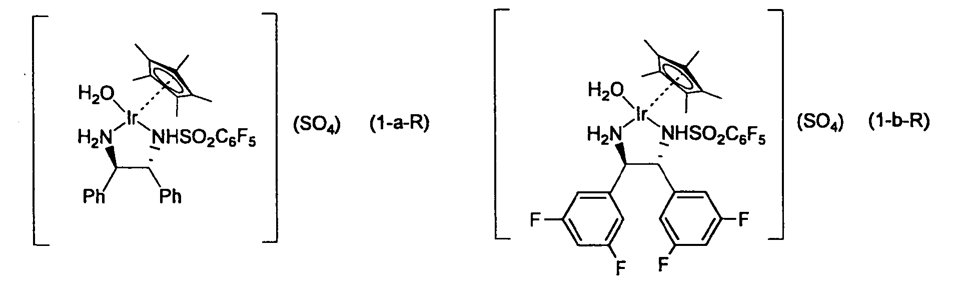

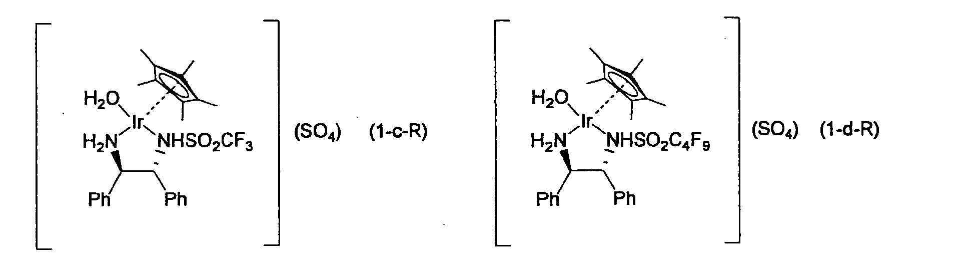

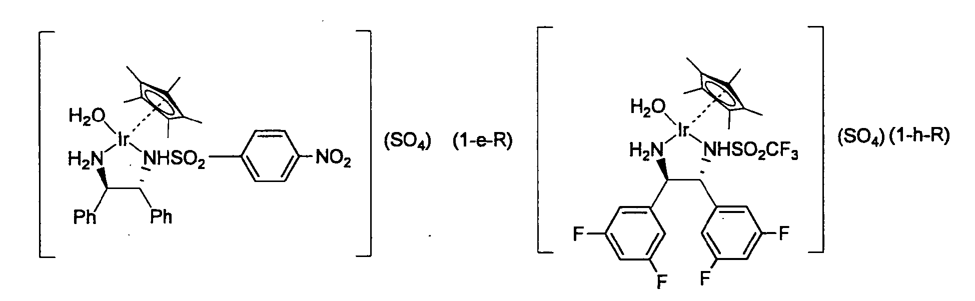

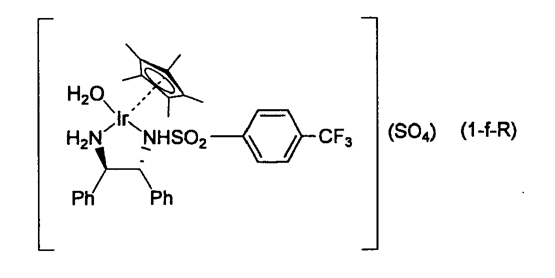

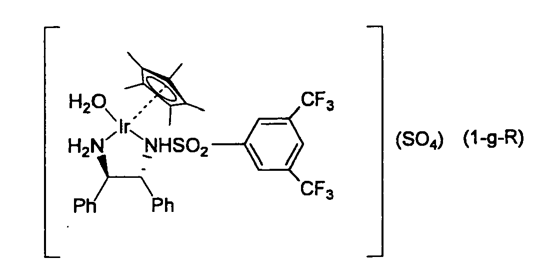

and (1-a-S) to (1-h-S):

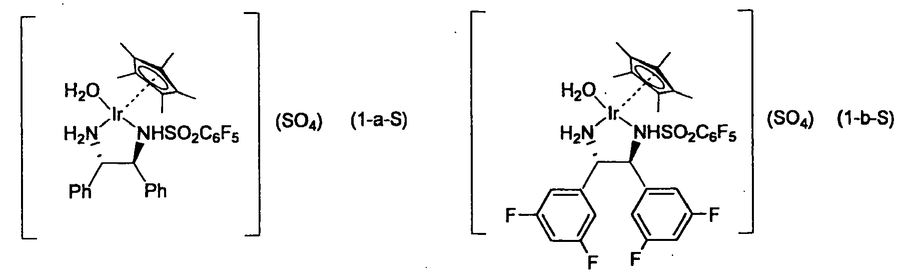

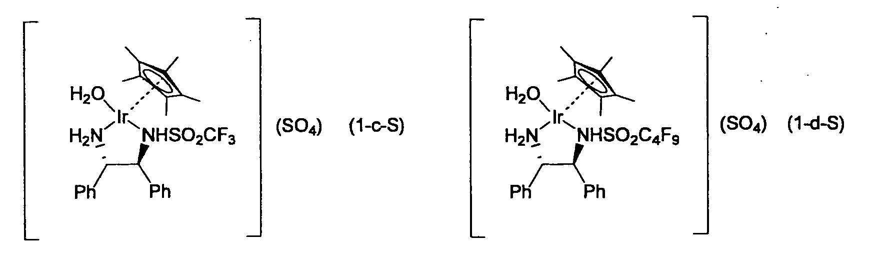

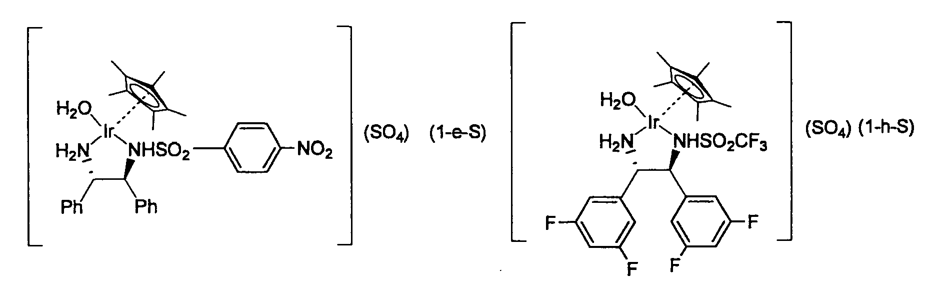

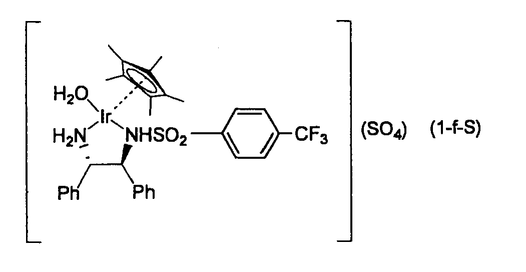

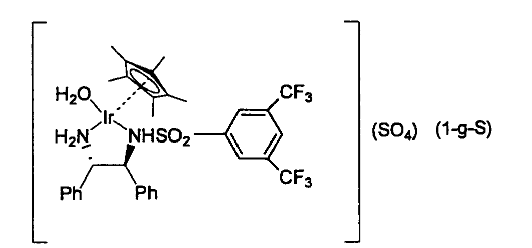

are preferable.

Since the stereoselectivity in an asymmetric transfer hydrogenation is extremely high, chiral iridium aqua complexes (1-a), (1-b) and (1-h) :

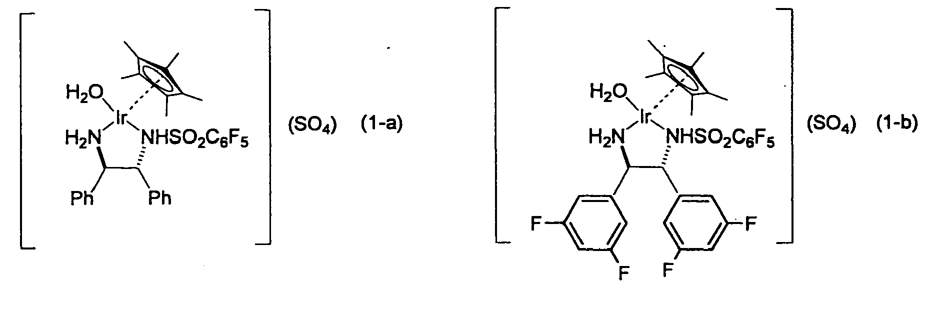

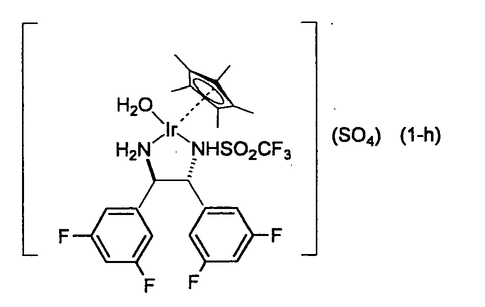

are preferable. That is, chiral iridium aqua complexes (1-a-R), (1-b-R) and (1-h-R) :

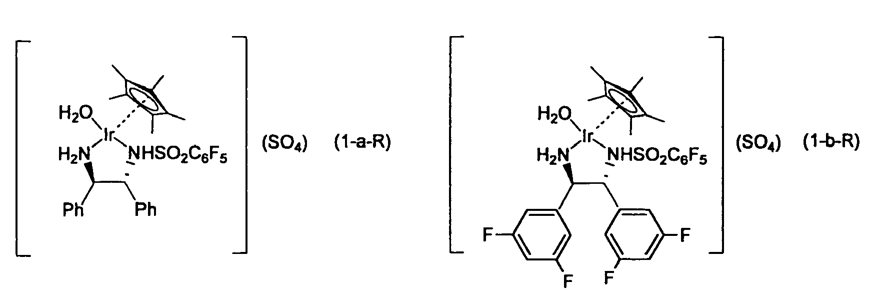

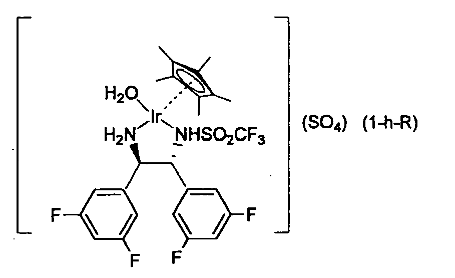

and chiral iridium aqua complexes (1-a-S), (1-b-S) and (1-h-S):

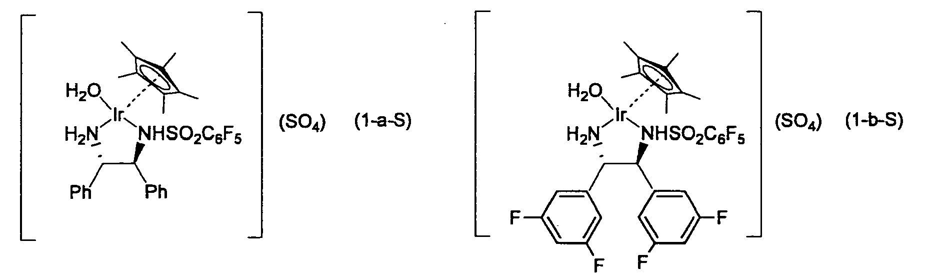

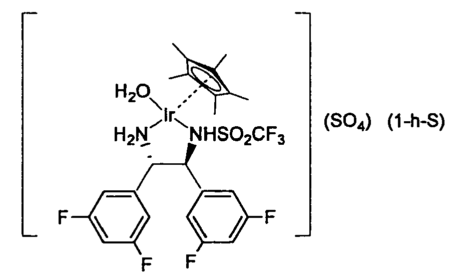

are preferable.

Chiral iridium aqua complex (1A) of the present invention can be produced by reacting iridium complex (2) with chiral diamine (3).

Iridium complex (2) can be produced according to the method described in Organometallics 1999, 18, 5470-5474.

Preferable specific examples of chiral diamine (3) include

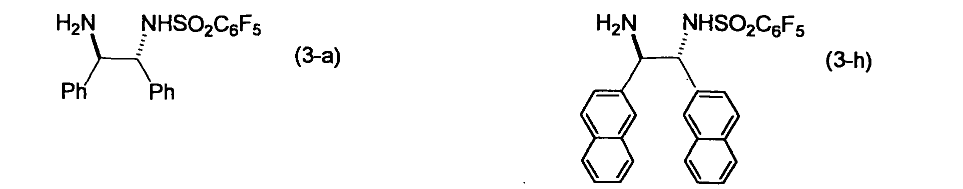

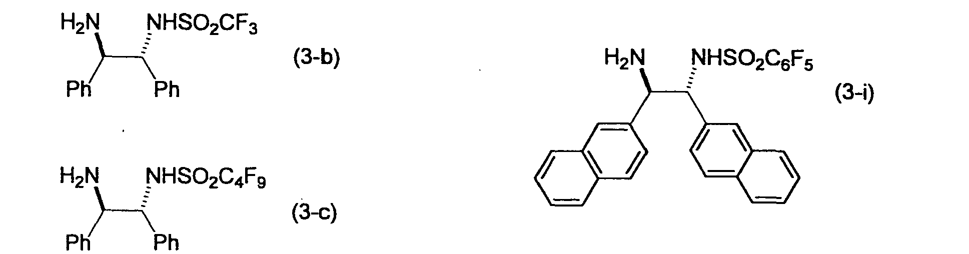

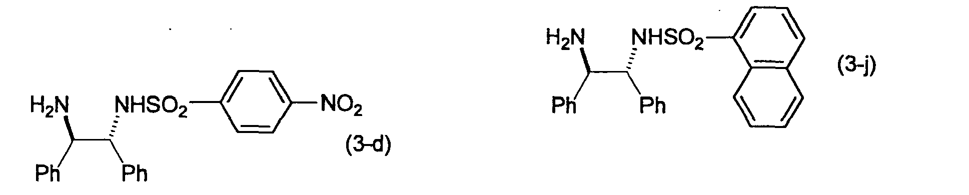

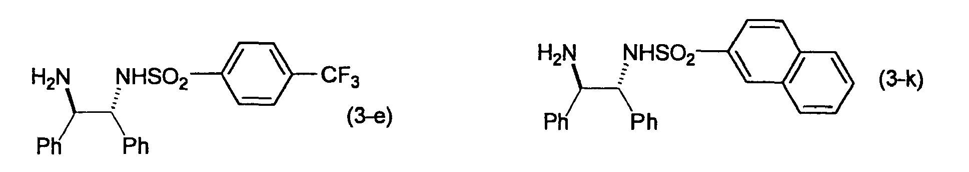

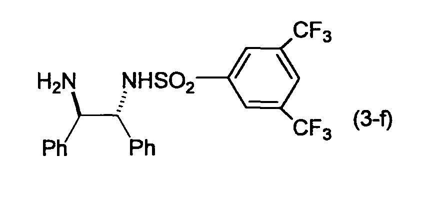

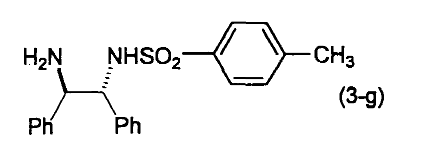

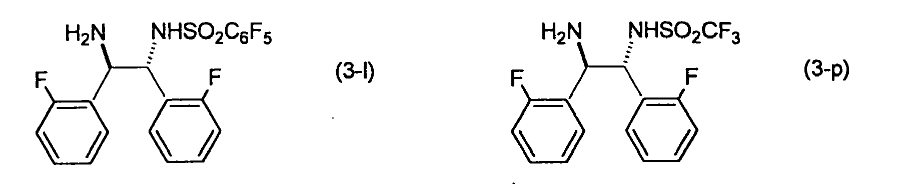

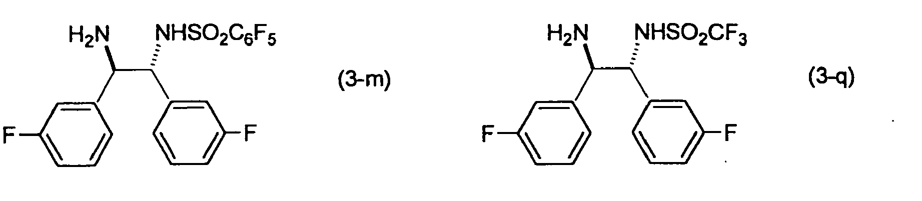

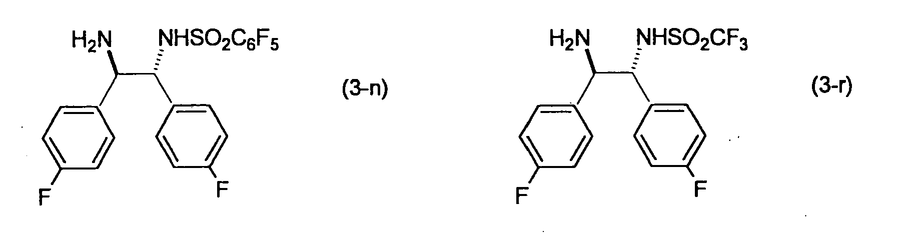

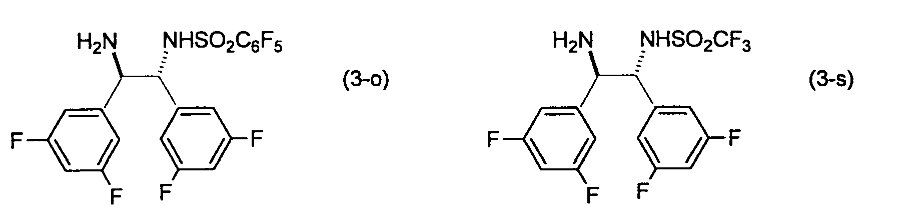

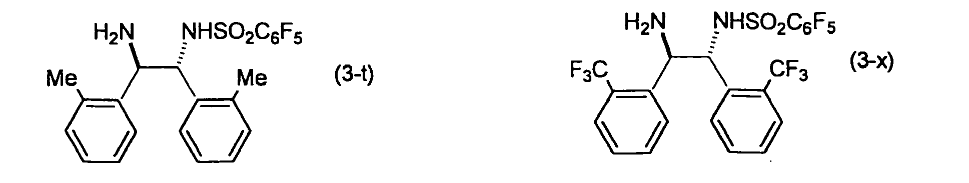

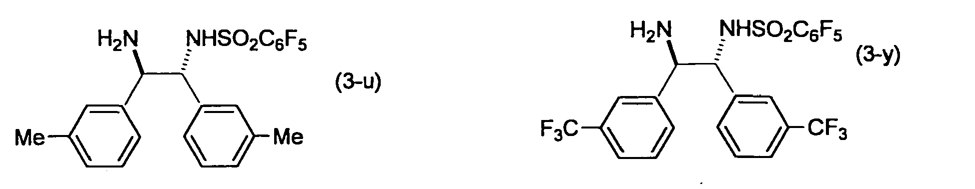

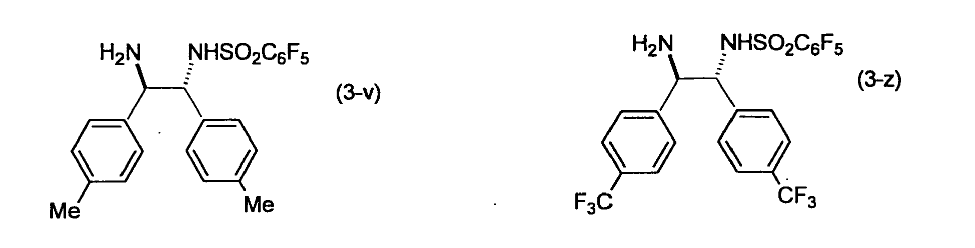

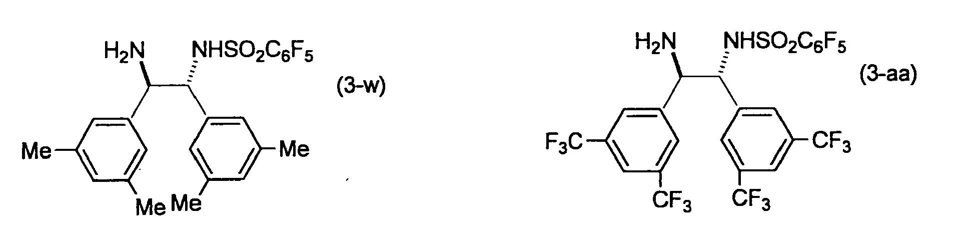

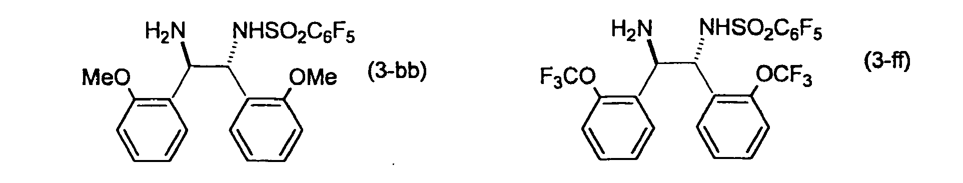

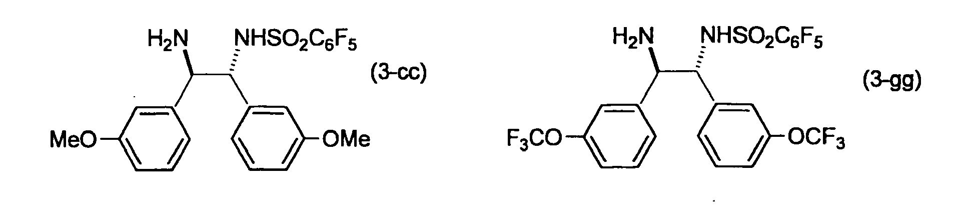

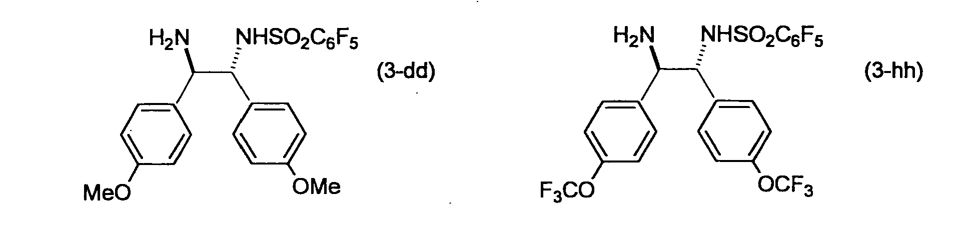

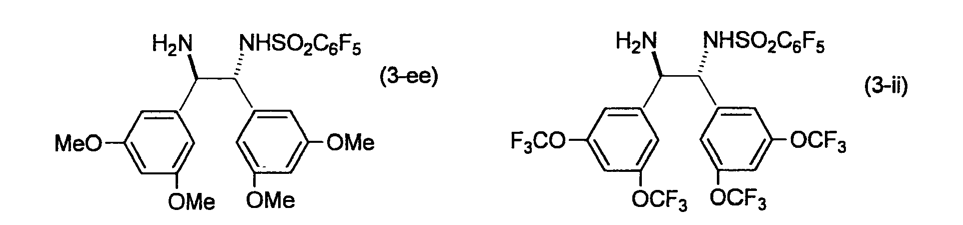

and the like. That is, the following R-forms

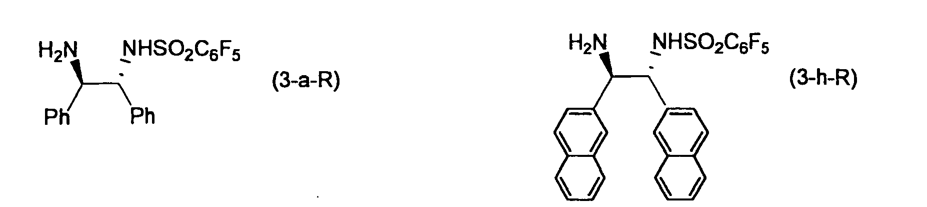

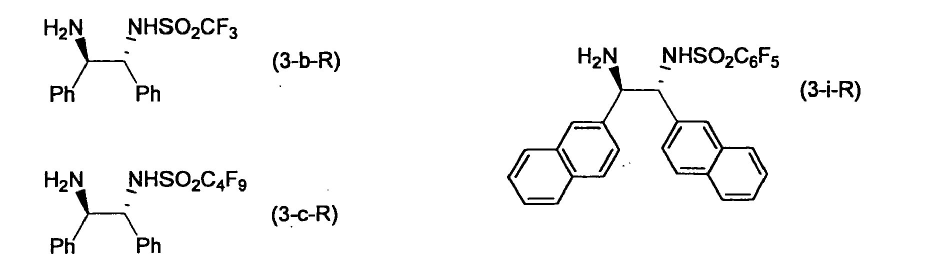

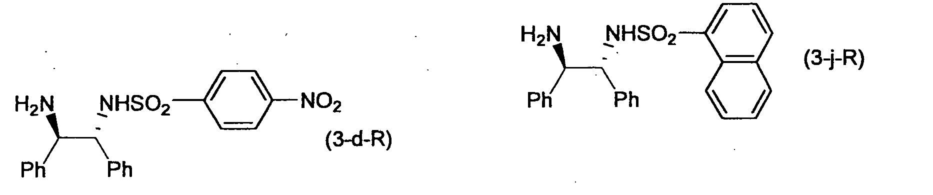

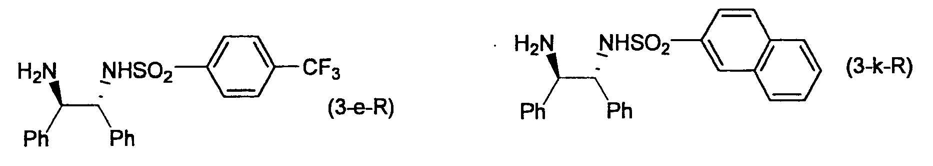

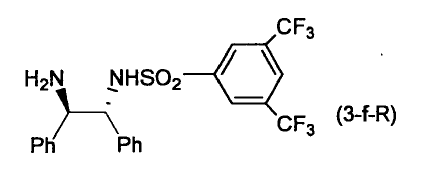

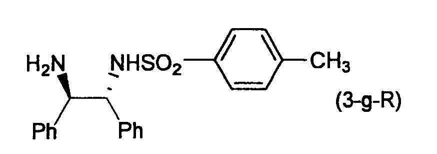

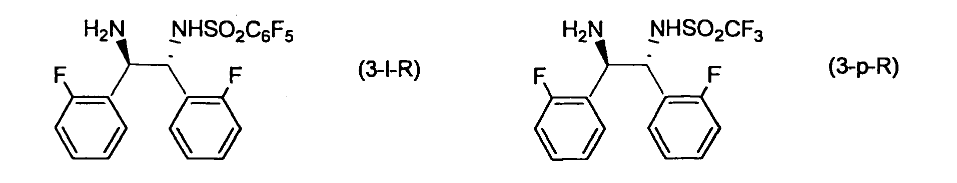

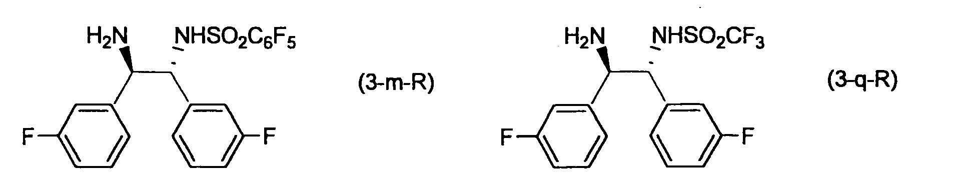

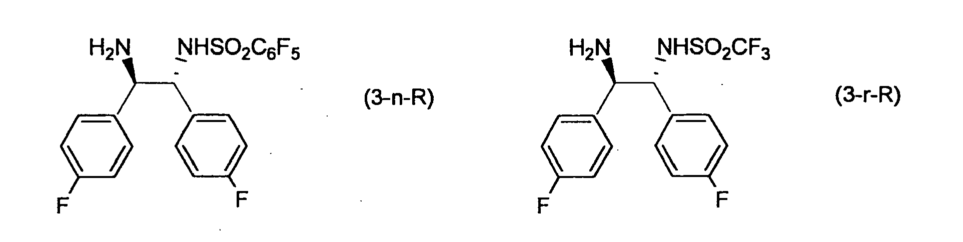

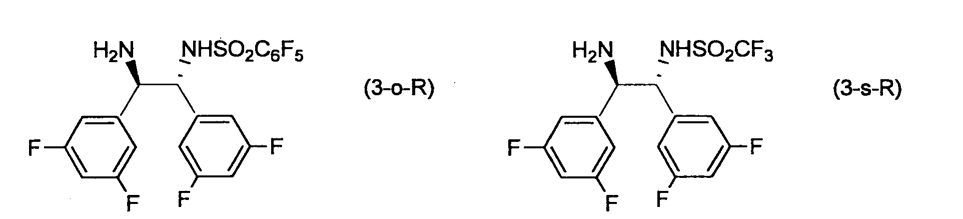

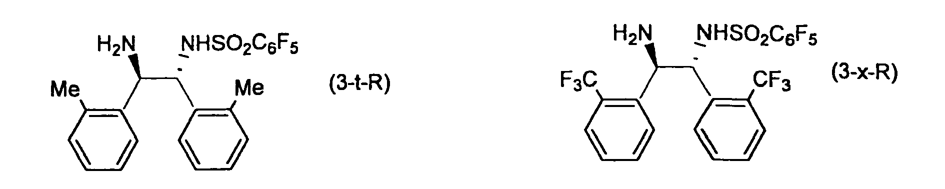

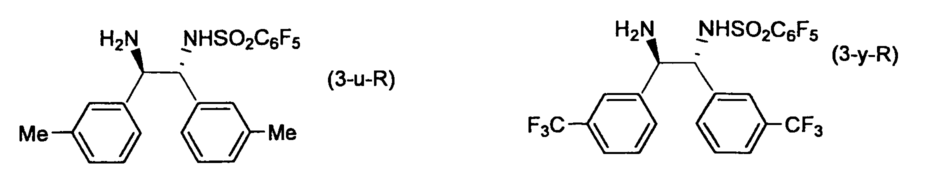

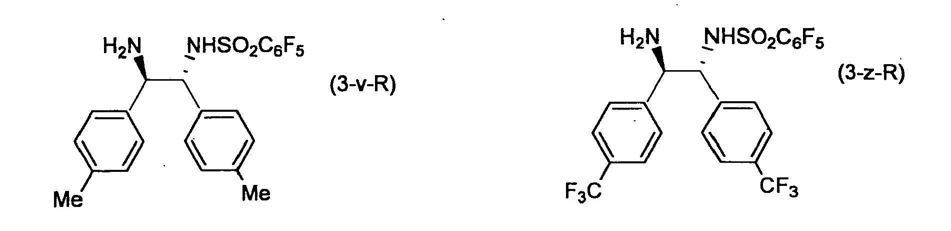

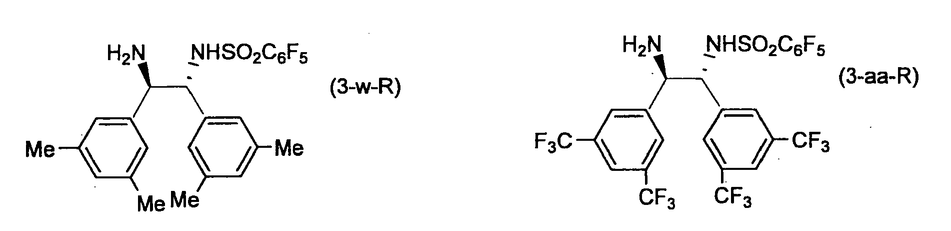

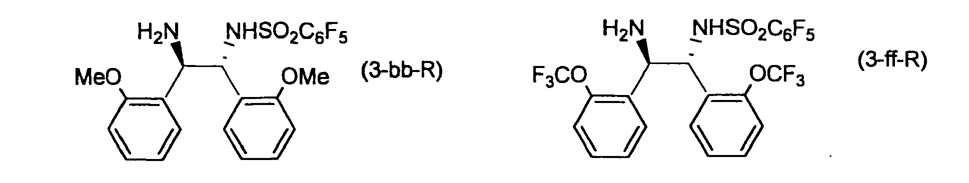

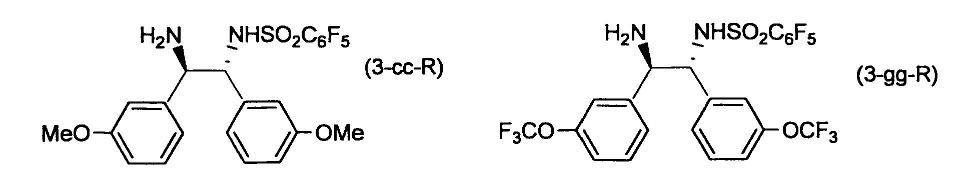

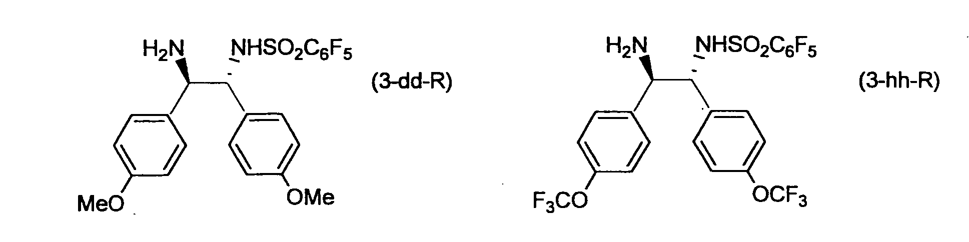

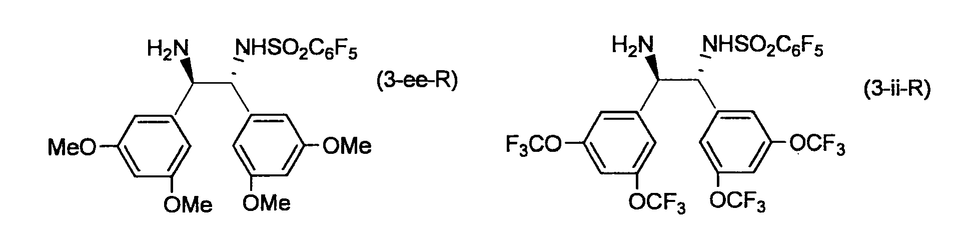

and the following S-forms

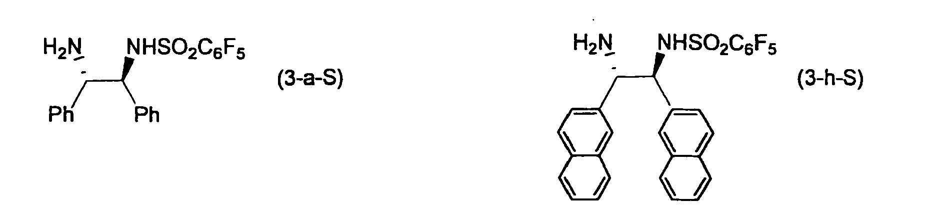

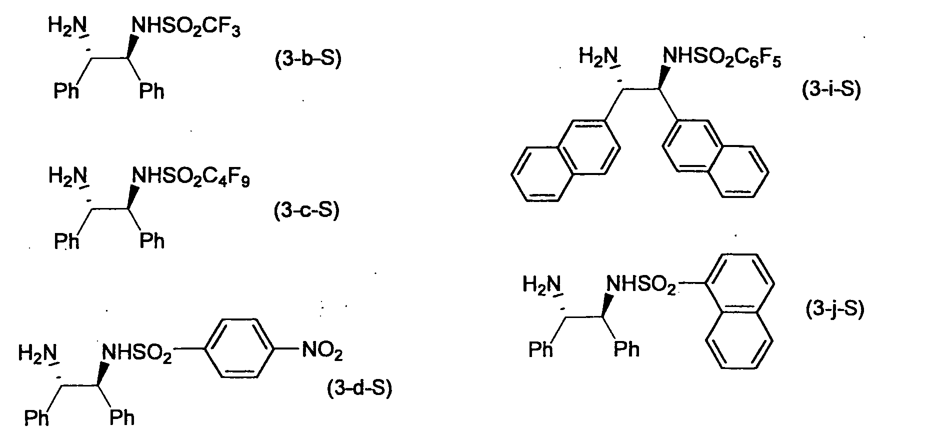

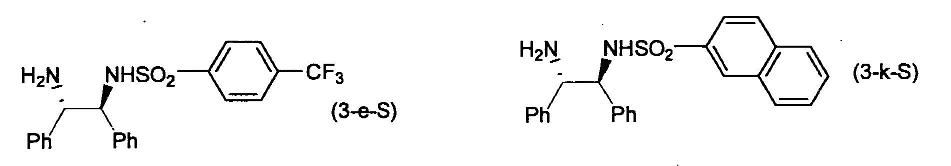

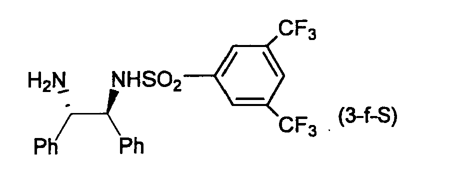

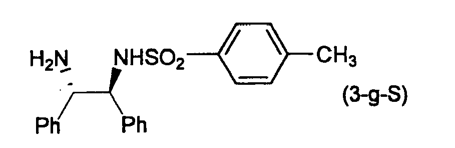

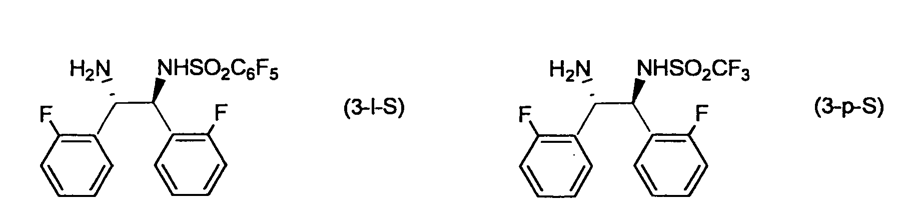

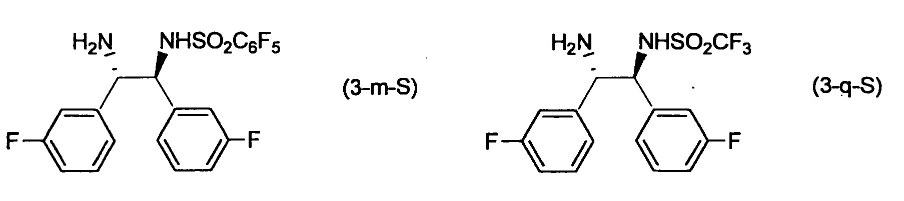

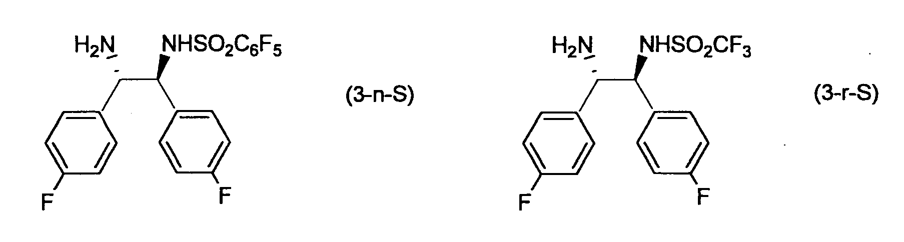

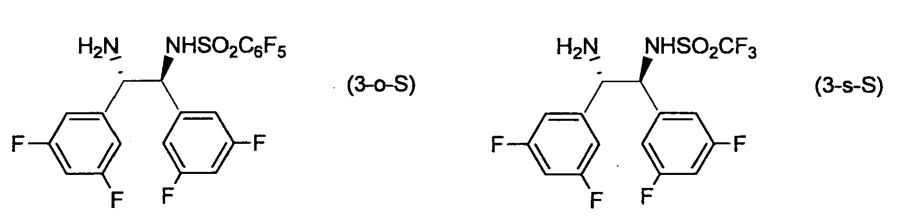

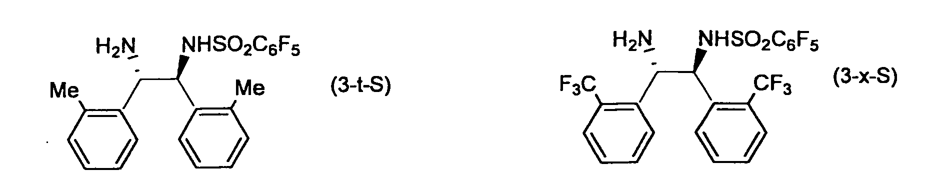

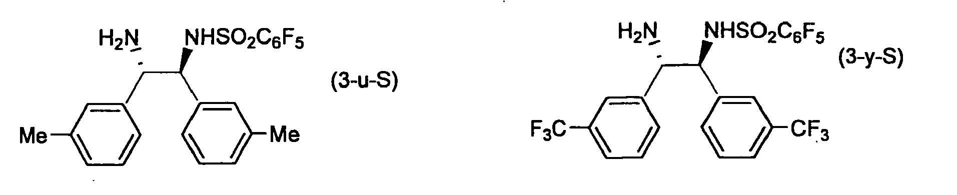

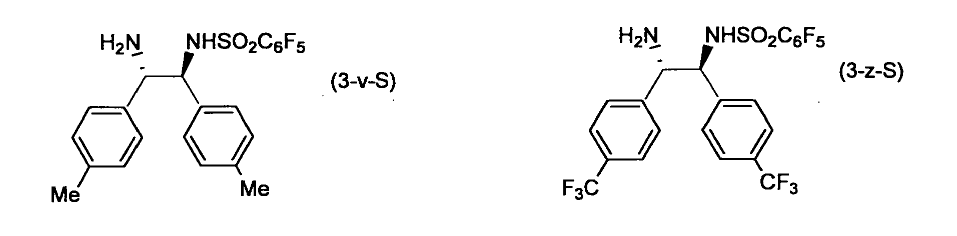

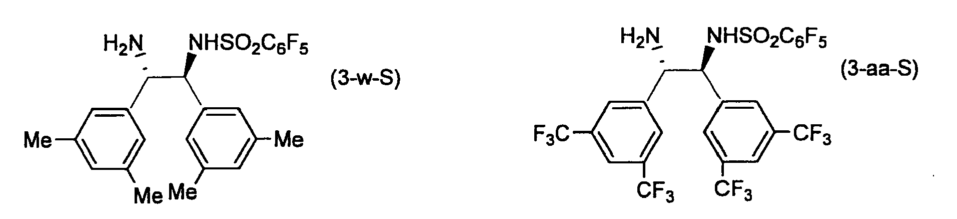

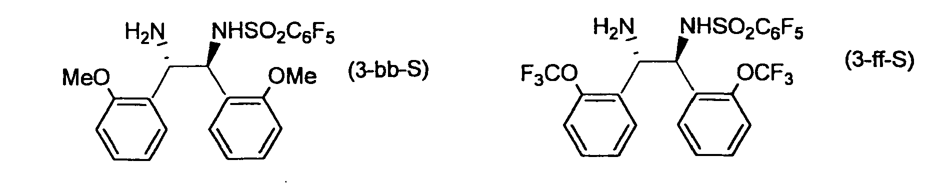

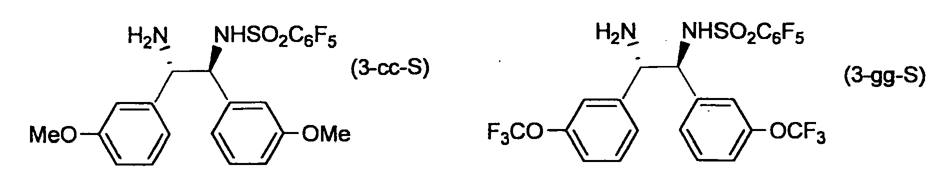

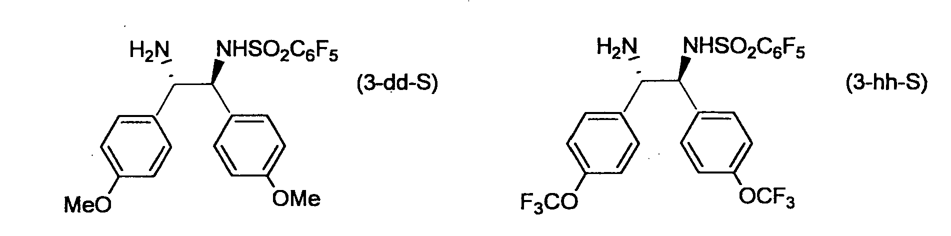

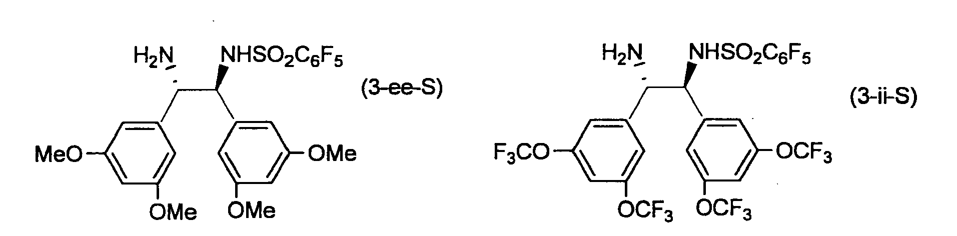

and the like are preferable. Among them, the following

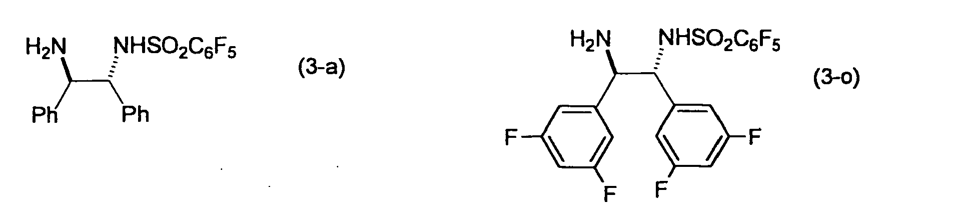

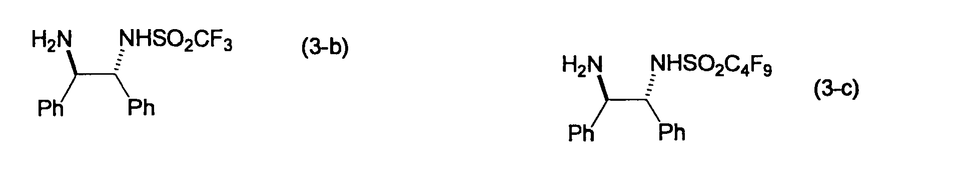

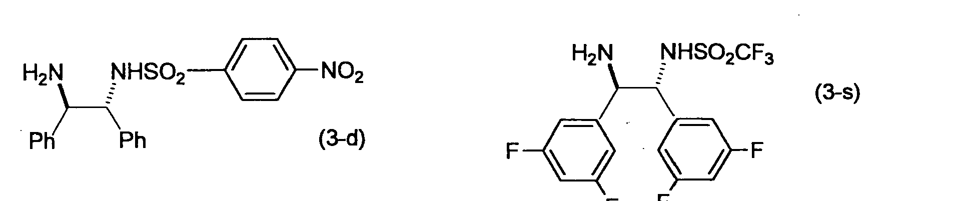

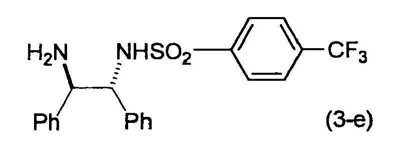

and

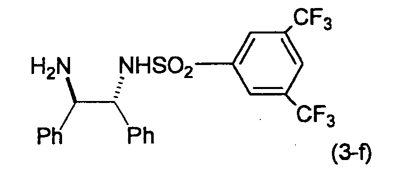

are preferable from the aspects of obtaining a chiral iridium aqua complex having high stereoselectivity by an asymmetric transfer hydrogenation. That is, the following R-forms

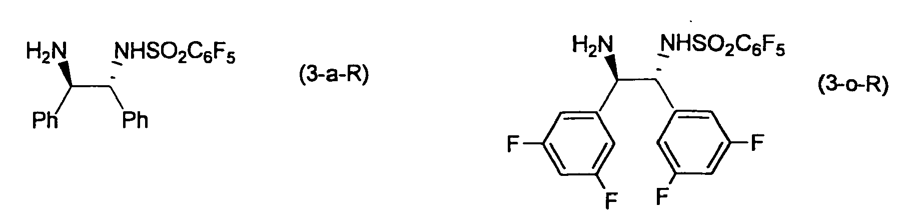

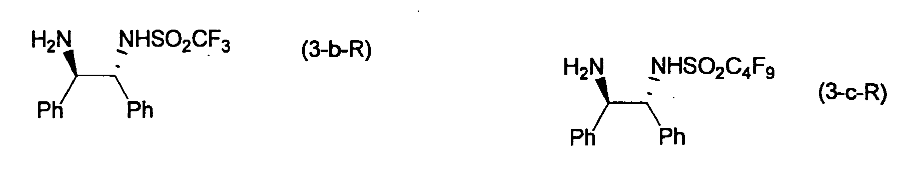

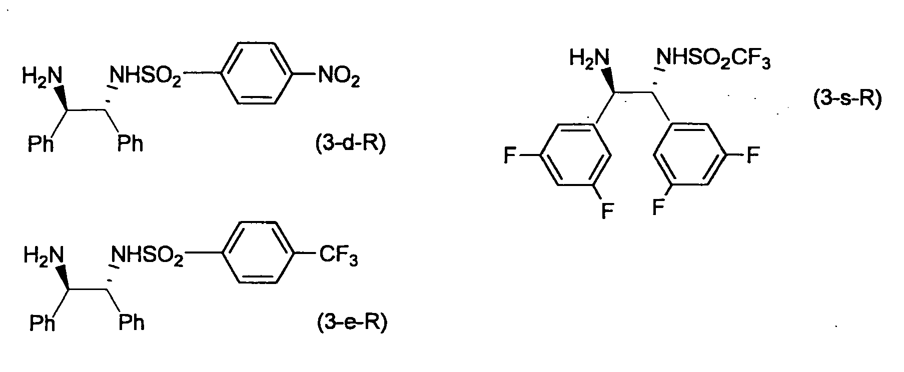

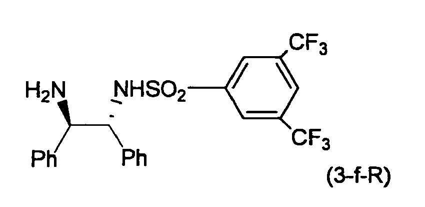

and the following S-forms

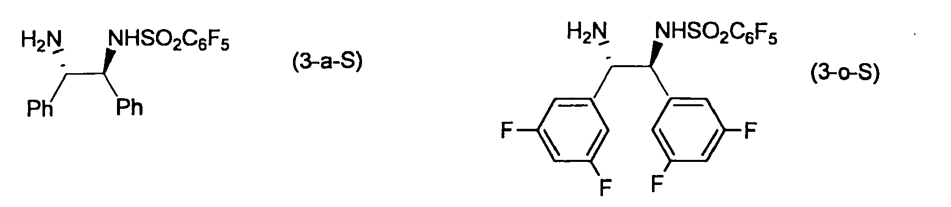

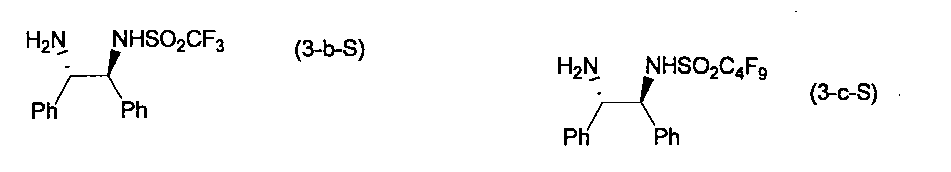

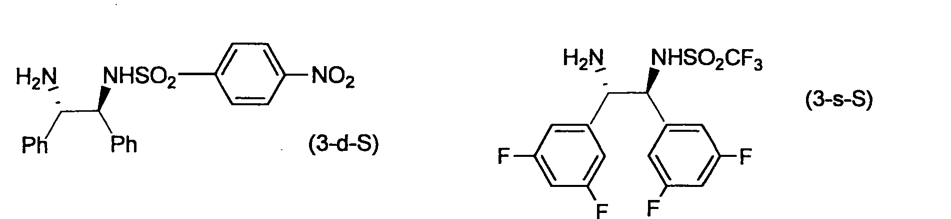

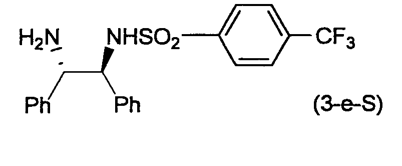

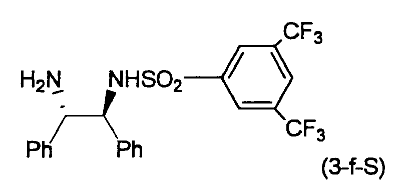

are preferable. Among them,

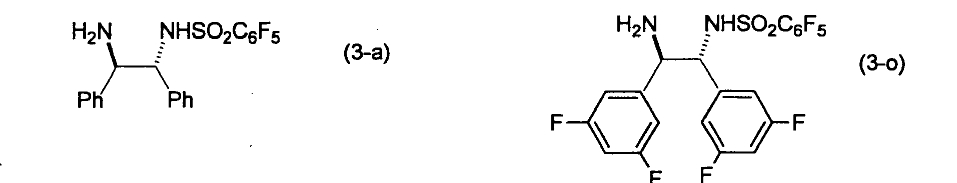

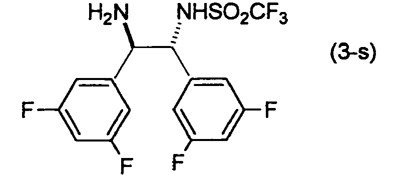

and

are particularly preferable. That is, the following R-forms[0132]

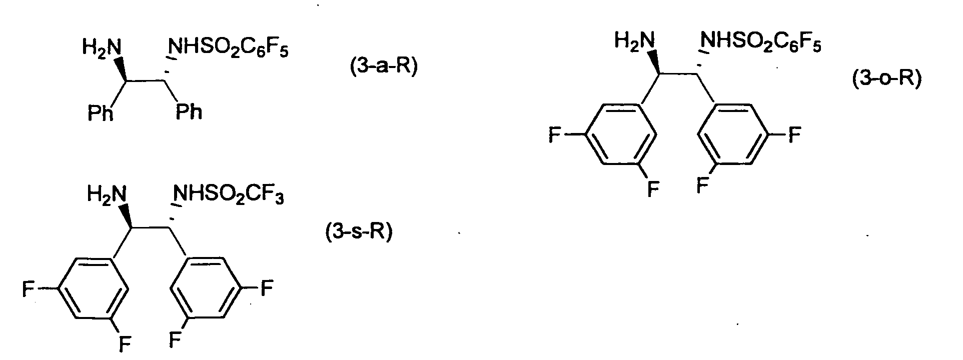

and the following S-forms

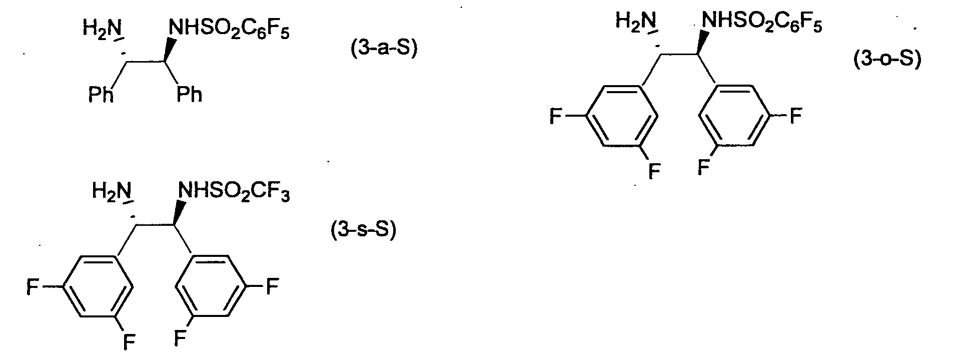

are particularly preferable.

Among the above-mentioned chiral diamine,

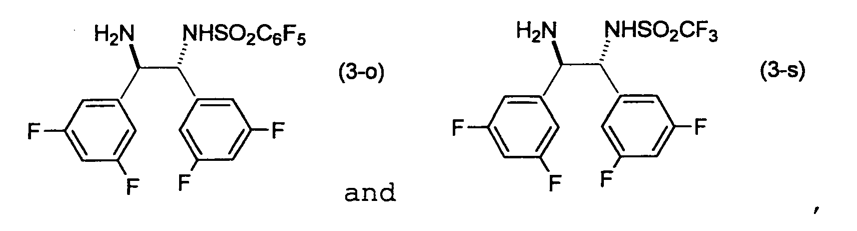

that is, the following R-forms

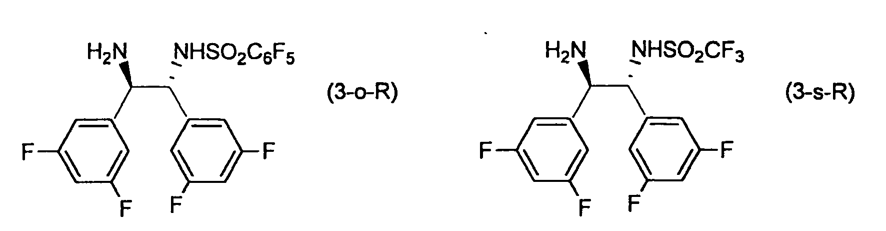

and the following S-forms

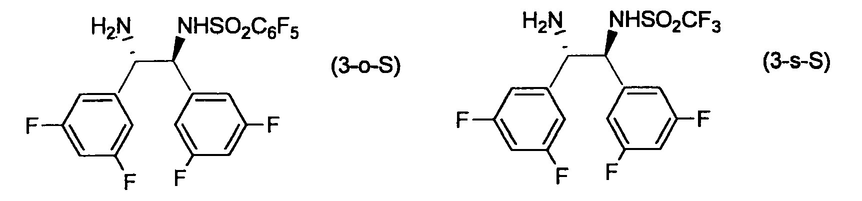

are novel.

The above-mentioned chiral diamine (3) which is commercially available, or is produced according to a known method (e.g., Chem. Commun., 2001, 2572-2573; J. Org. Chem. 2004, 69, 5187-5195) or an analogous method thereto can be used.

The above-mentioned chiral diamine (3) is not a racemate (1:1 mixture of trans forms thereof). The enantiomer excess of chiral diamine (3) is any value of more than 0% ee and not more than 100% ee, preferably not less than 90% ee, particularly not less than95% ee.

In the above-mentioned reaction, the amount of chiral diamine (3) to be used is generally 0.8 to 2.0 mol, preferably 0.9 to 1.2 mol from the aspects of economic efficiency, per 1 mol of iridium complex (2).

The reaction is carried out according to the method described in Organometallics, 2001, 20, 4903. To be specific, the reaction is carried out by mixing iridium complex (2) and chiral diamine (3) in a solvent as shown in the following.

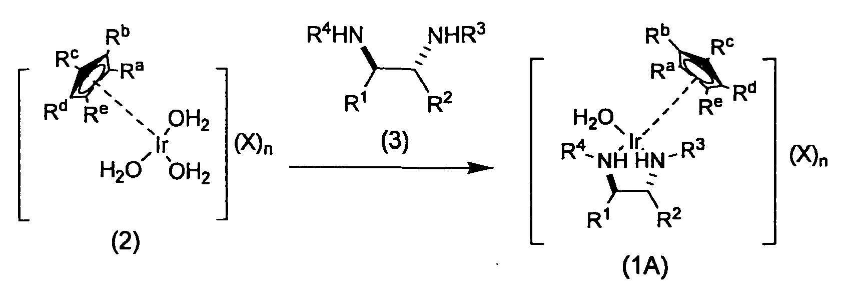

Examples of the solvent include water, alcohols such as methanol and the like, mixtures thereof and the like. Among them, water, and a mixed solvent of water-alcohol are preferable. While the content ratio (volume ratio) of water and the alcohol in the mixed solvent varies depending on the kind of chiral diamine, it is generally 1:1 to 10:1, preferably 1:1 to 5:1. The amount of the solvent to be used is generally 3 to 100 mL, preferably 10 to 65 mL from the aspects of operability and economic efficiency, per 1 g of iridium complex (2).

The reaction temperature is generally 0 to 110°C, preferably 5 to 50°C. While the reaction time varies depending on the kind of chiral diamine (3), it is generally 1 to 50 hours, preferably 1 to 24 hours.
After completion of the reaction, the reaction mixture is concentrated to give chiral iridium aqua complex (1A). Where necessary, chiral iridium aqua complex (1A) can be purified by operation such as recrystallization and the like.

Chiral iridium aqua complex (1A) of the present invention has good stability to air and water.

Chiral iridium aqua complex (1A) of the present invention is a catalyst suitable for an asymmetric transfer hydrogenation, particularly an asymmetric transfer hydrogenation of carbonyl compound (4). For example, it can be preferably used for producing optically active hydroxy compound (5) by subjecting carbonyl compound (4) to an asymmetric transfer hydrogenation. In addition, chiral iridium aqua complex (1A) is also a catalyst suitable for an asymmetric transfer hydrogenation of nitroolefin compound (6). For example, it can be preferably used for producing optically active nitroalkane compound (7) by subjecting nitroolefin compound (6) to an asymmetric transfer hydrogenation.

### Asymmetric transfer hydrogenation of carbonyl compound (4)

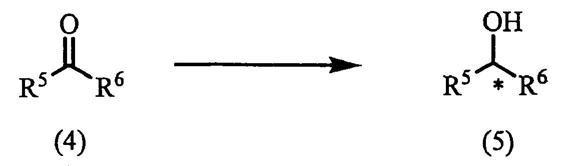

In the reaction, the amount of chiral iridium aqua complex (1A) to be used is generally 0.001 to 0.1 mol, preferably 0.002 to 0.05 mol from the aspects of reactivity and economic efficiency, per 1 mol of carbonyl compound (4).
The reaction is generally carried out in a solvent, in the presence of a hydrogen-donor compound. The reaction is carried out by adding carbonyl compound (4) to a solution of chiral iridium aqua complex (1A), and then stirring the mixture.
Preferable examples of the hydrogen-donor compound include formic acid or a salt thereof. From the aspects of conversion, formic acid is particularly preferable. The amount of the hydrogen-donor compound to be used is generally 1 to 100 mol, preferably 2 to 10 mol from the aspects of economic efficiency, per 1 mol of carbonyl compound (4).
When formic acid is used as a hydrogen-donor compound, it may be a solvent.
Examples of the solvent include water, alcohols such as methanol and the like, mixtures thereof and the like. Among them, water, and a mixed solvent of water-alcohol are preferable.
While the content ratio (volume ratio) of water and the alcohol in the mixed solvent varies depending on the kind and amount of carbonyl compound (4) as a starting material and chiral iridium aqua complex (1A), it is generally 10:1 to 1:10, preferably 3:1 to 1:3. The amount of the solvent to be used is generally 1 to 100 mL, preferably 3 to 50 mL from the aspects of operability and economic efficiency, per lg of carbonyl compound (4).

From the aspects of reactivity, the reaction is preferably carried out under the condition of pH 2 to 5, more preferably pH 2.0 to 4.5, particularly preferably pH 2.0 to 3.5. The optimal range of the pH varies depending on the kind of carbonyl compound (4) (particularly R⁶). For example, the optimal pH for carbonyl compound (4) wherein R⁶ is cyanomethyl, azidomethyl, chloromethyl group or the like is 3.5, and the optimal pH for carbonyl compound (4) wherein R⁶ is nitromethyl group or the like is 2. The pH is mainly adjusted by addition of a base (e.g., sodium hydroxide etc.).

The reaction temperature is generally 30 to 100°C, preferably 40 to 85°C. While the reaction time varies depending on the kind of carbonyl compound (4), it is generally 1 to 50 hours, preferably 1 to 24 hours.

Chiral iridium aqua complex (1A) for the reaction is particularly preferably

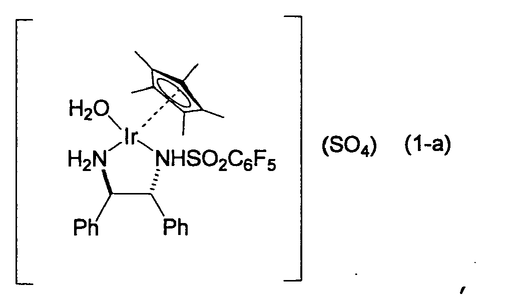

that is,

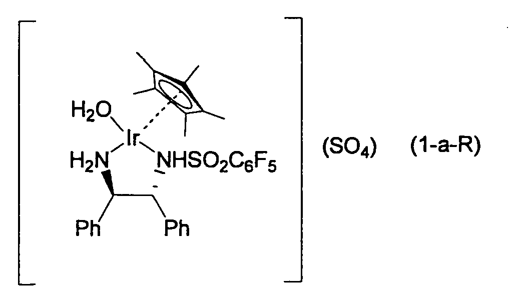

or

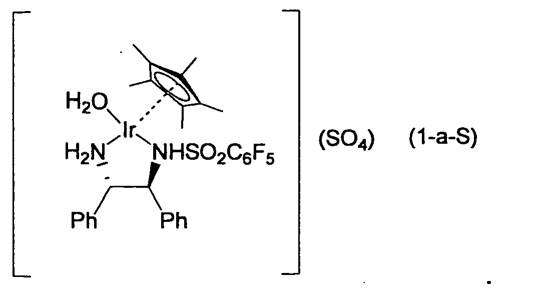

Optically active hydroxy compound (5) is obtained by the asymmetric transfer hydrogenation using chiral iridium aqua complex (1A) of the present invention. The steric configuration of the optically active hydroxy compound (5) depends on the steric configuration of the chiral iridium aqua complex (1A), and whether it is R-configuration or S-configuration depends on the kind of the chiral iridium aqua complex (1A).

The thus-obtained optically active hydroxy compound (5) can be isolated by subjecting the reaction mixture to a conventional posttreatment (e.g., neutralization, extraction, washing with water, distillation, crystallization etc.). In addition, optically active hydroxy compound (5) can be purified by a purification means such as recrystallization, extraction purification, distillation, adsorption treatment with activated carbon, silica, alumina and the like, chromatography method (e.g., silica gel column chromatography etc.). It can also be used for the next step without particular purification; for example, an extraction solution or a residue after solvent evaporation can be directly provided for the next step.

The reaction using chiral iridium aqua complex (1A) of the present invention as a catalyst can achieve higher stereoselectivity (not less than 80% ee, particularly not less than 95% ee) and higher yield, as compared to conventional iridium complexes. Particularly, carbonyl compound (4) wherein R⁵ is phenyl, halogen-substituted phenyl, C₁₋₆ alkyl-substituted phenyl, C₁₋₆ alkoxy-substituted phenyl, cyano-substituted phenyl, naphthyl, furyl or thienyl, and R⁶ is cyanomethyl, nitromethyl, chloromethyl or azidomethyl can be obtained with higher stereoselectivity (not less than 80% ee, particularly not less than 95% ee) in a higher yield, by the reduction reaction.

Asymmetric transfer hydrogenation of nitroolefin compound (6)

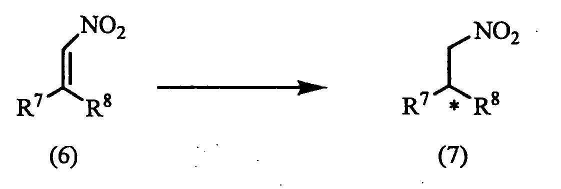

In the reaction, the amount of chiral iridium aqua complex (1A) to be used is generally 0.001 to 0.1 mol, preferably 0.003 to 0.01 mol from the aspects of reactivity and economic efficiency, per 1 mol of nitroolefin compound (6).
The reaction is generally carried out in a solvent, in the presence of a hydrogen-donor compound. The reaction is carried out by adding nitroolefin compound (6) to a solution of chiral iridium aqua complex (1A), and then stirring the mixture.
Preferable examples of the hydrogen-donor compound include formic acid or a salt thereof. From the aspects of conversion, formic acid is particularly preferable. The amount of the hydrogen-donor compound to be used is generally 1 to 100 mol, preferably 2 to 10 mol from the aspects of economic efficiency, per 1 mol of nitroolefin compound (6).
When formic acid is used as a hydrogen-donor compound, it may be a solvent.
Examples of the solvent include water, alcohols such as methanol and the like, mixtures thereof and the like. Among them, water, and a mixed solvent of water-alcohol are preferable. While the content ratio (volume ratio) of water and the alcohol in the mixed solvent varies depending on the kind and amount of nitroolefin compound (6) as a starting material and chiral iridium aqua complex (1A), it is generally 10:1 to 1:10, preferably 3:1 to 1:3. The amount of the solvent to be used is generally 1 to 100 mL, preferably 3 to 50 mL from the aspects of operability and economic efficiency, per lg of nitroolefin compound (6).

From the aspects of reactivity and selectivity, the reaction is preferably carried out under the condition of pH 2 to 5, more preferably pH 2.0 to 4.5, particularly preferably pH 2.0 to 3.5. The pH is mainly adjusted by addition of a base (e.g., sodium hydroxide etc.).

The reaction temperature is generally 30 to 100°C, preferably 40 to 85°C. While the reaction time varies depending on the kind of nitroolefin compound (6), it is generally 1 to 50 hours, preferably 1 to 24 hours.

Chiral iridium aqua complex (1A) for the reaction is particularly preferably

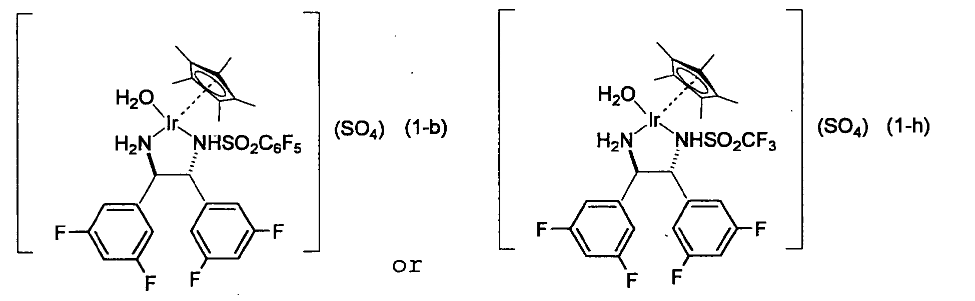

that is,

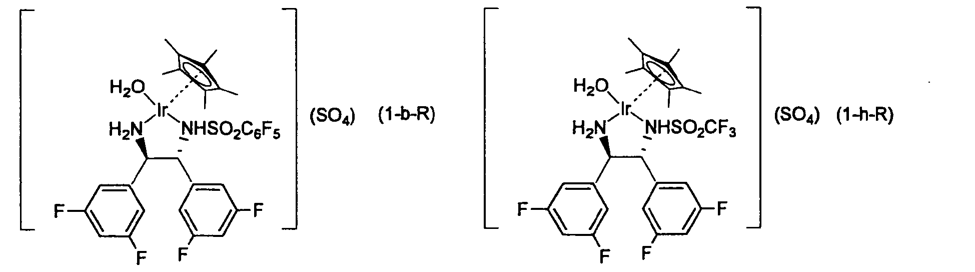

or

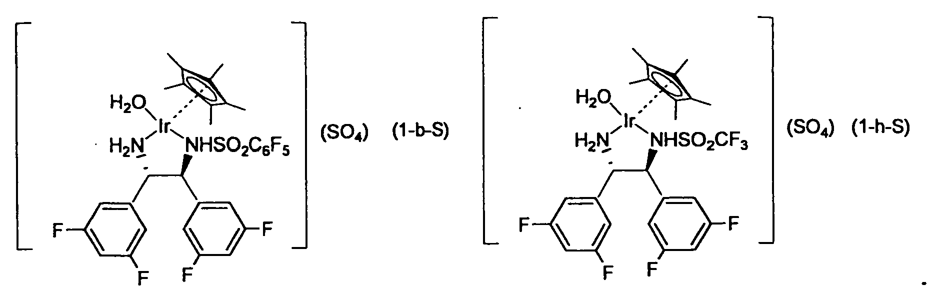

Optically active nitroalkane compound (7) is obtained by the asymmetric transfer hydrogenation using the chiral iridium aqua complex (1A) of the present invention. The steric configuration of the optically active nitroalkane compound (7) depends on the steric configuration of chiral iridium aqua complex (1A), and whether it is R-configuration or S-configuration depends on the kind of the chiral iridium aqua complex (1A).

Thus-obtained optically active nitroalkane compound (7) can be isolated by subjecting the reaction mixture to a conventional posttreatment (e.g., neutralization, extraction, washing with water, distillation, crystallization etc.). In addition, optically active nitroalkane compound (7) can be purified by a purification means such as recrystallization, extraction purification, distillation, adsorption treatment with activated carbon, silica, alumina and the like, chromatography method (e.g., silica gel column chromatography etc.). It can also be used for the next step without particular purification; for example, an extraction solution or a residue after solvent evaporation can be directly provided for the next step.

The reaction using chiral iridium aqua complex (1A) of the present invention as a catalyst can achieve higher stereoselectivity (not less than 80% ee, particularly not less than 95% ee) and higher yield, as compared to conventional iridium complexes. Particularly, nitroolefin compound (6) wherein R⁷ is phenyl and R⁸ is methyl can be obtained in a higher stereoselectivity (not less than 80% ee, particularly not less than 95% ee) in a higher yield, by the reduction reaction.

Since chiral iridium aqua complex (1A) of the present invention has good solubility in water, the asymmetric transfer hydrogenation can be carried out in an aqueous solvent which is environmentally friendly and suitable for green chemistry. Even if the asymmetric transfer hydrogenation is carried out in the presence of formic acid or a salt thereof, it can be performed safely since the danger caused by hydrogen generation and the like is absent.

### Examples

The present invention is explained more specifically in the following by referring to Examples, by which the invention is not to be limited. Cp^{*} means η⁵-pentamethylcyclopentadienyl anion.

### Reference Example 1 Production of [Cp^{*}Ir(H₂O)₃] (SO₄)

According to the method described in Organometallics 1999, 18, 5470, a mixture of Ag₂SO₄ (3.36 mmol, 1.05 g) and [Cp^{*}IrCl₂]₂ (1.68 mmol, 1.34 g) in water (12 mL) was stirred at room temperature for 12 hours, and filtered to remove AgCl. The solvent of the filtrate was evaporated under reduced pressure to give the object (1.55 g, 97%) as a yellow solid.
¹H NMR (300 MHz, D₂O δ: 1.59 (s, 15 H).

### Example 1 Production of iridium aqua complex (1-a-R)

A solution of [Cp^{*}Ir(H₂O)₃] (SO₄) (0.11 mmol, 53 mg) and diamine (3-a-R) (0.11 mmol, 49 mg) in a mixed solvent of water:methanol (1:1 (volume ratio), 2 mL) was stirred at room temperature for 1 hour.
The solvent was evaporated under reduced pressure to give the object as red powder.
¹H-NMR (300 MHz, CD₃OD) δ: 1.89 (s, 15H), 4.22 (d, J = 3.9 Hz, 1H), 4.66 (d, d, J = 4.2 Hz, 1H), 7.16-7.31 (m, 10H).
¹³C-NMR (75 MHz, CD₃OD) δ: 10.4, 69.2, 76.2, 92.2, 127.5, 128.3, 129.1, 129.2, 138.4, 140.2.
HR-MALDI calcd for C₃₀H₂₉IrN₂O₂S [M-SO₄-H₂O]⁺ 769.1499, found 769.1479.
[α]^{D}₂₆ -242.22 (c 1.0, CHCl₃).

In the same manner as in Example 1 and using the corresponding chiral diamine, iridium aqua complex (1-b-S), iridium aqua complex (1-c-R), iridium aqua complex (1-d-R), iridium aqua complex (1-e-R), iridium aqua complex (1-f-R), iridium aqua complex (1-g-R) and iridium aqua complex (1-h-S) were obtained.

### iridium aqua complex (1-b-S)

¹H-NMR (300 MHz, CD₃OD) δ: 1.87 (s, 15H), 4.27-4.31 (m, 1H), 4.64-4.66 (m, 1H), 6.87-6.98 (m, 6H).
HR-MALDI calcd for C₃₀H₂₅IrN₂O₂S [M-SO₄-H₂O]⁺ 841.1122, found 841.1102.
[α]^{D}₂₆ -206.25 (c 0.50, CHCl₃).

### iridium aqua complex (1-c-R)

¹H-NMR (300 MHz, CD₃OD) δ: 1.80 (s, 15H), 4.15 (d, J = 6.9 Hz, 1H), 4.74 (d, J = 7.2 Hz, 1H), 7.06-7.27 (m, 10H).
¹³C-NMR (75 MHz, CD₃OD) δ: 9.86, 71.0, 71.2, 90.5, 127.8, 128.2, 129.0, 129.3, 130.1.
HR-MALDI calcd for C₂₅H₂₉IrN₂O₂S [M-SO₄-H₂O]⁺ 671.1531, found 671.1514.
[α]^{D}₂₈ -293.16 (c 0.25, CH₃OH).

### iridium aqua complex (1-h-S)

¹H-NMR (300 MHz, CD₃OD) δ: 1.82 (s, 15H), 4.38 (d, J = 4.8 Hz), 4.86 (d, J = 4.8 Hz), 6.89-6.98 (m, 5H), 6.99-7.03 (m, 2H). HR-MALDI calcd for C₂₅H₂₅IrN₂O₂S [M-SO₄-H₂O]⁺ 743.1154, found 743.1147.
[α]^{D}₂₆ 18.8 (c 0.25, CH₃OH).

### Example 2 Asymmetric transfer hydrogenation of 2-cyanoacetophenone

Iridium aqua complex shown in Table 1 (the amount shown in Table 1 (mol%) relative to 2-cyanoacetophenone), 2-cyanoacetophenone (0.5 mmol) and 1.0 M formic acid aqueous solution (2.5 mL, 5 eq, adjusted to pH=3.5 with 4M sodium hydroxide) were mixed at room temperature, and the mixture was stirred. After confirming the disappearance of 2-cyanoacetophenone by TLC, the mixture was extracted with dichloromethane, and the organic layer was dried over anhydrous Na₂SO₄, and concentrated. The residue was purified by silica gel column chromatography to give (S)-3-phenyl-3-hydroxypropionitrile. The conversion and ee were shown in Table 1.

**Table 1**

| | iridium aqua complex | amount of use [mol%] | conversion [%] | ee [%] |
|---|---|---|---|---|
| 2-1 | (1-a-R) | 0.50 | 90 | 94 |
| 2-2 | (1-a-R) | 0.25 | quantitative | 94 |
| 2-3 | (1-c-R) | 0.25 | quantitative | 90 |
| 2-4 | (1-d-R) | 1 | 97 | 94 |

### Example 3

The following reaction was carried out using iridium aqua complex (1-a-R).

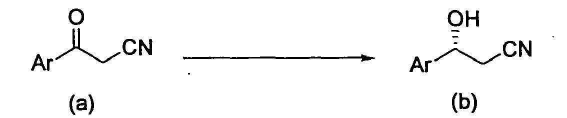

Iridium aqua complex (1-a-R) shown in Table 2 (the amount shown in Table 2 (mol%) relative to compound (a)), compound (a) (0.5 mmol) and 1.0 M formic acid aqueous solution (2.5 mL, 5 eq, adjusted to pH=3.5 with 4M sodium hydroxide) were mixed at room temperature, and the mixture was stirred. After confirming the disappearance of compound (a) by TLC, the mixture was extracted with dichloromethane, and the organic layer was dried over anhydrous Na₂SO₄, and concentrated. The residue was purified by silica gel column chromatography to give the corresponding compound (b). The conversion and ee were shown in Table 2.

**Table 2**

| | Ar | amount of use [mol%] | conversion [%] | ee [%] |
|---|---|---|---|---|
| 3-1 | Ph- | 0.25 | 96 | 94 |
| 3-2 | 3-F-Ph- | 0.50 | 95 | 91 |
| 3-3 | 3-Cl-Ph- | 0.25 | 90 | 90 |
| 3-4 | 3-Me-Ph- | 0.50 | 96 | 93 |
| 3-5 | 3-MeO-Ph- | 0.25 | 96 | 95 |
| 3-6 | 4-MeO-Ph- | 0.50 ^{a)} | 80 | - |
| 3-7 | 4-CN-Ph- | 0.25 | 97 | 86 |
| 3-8 | 2-naphthyl | 0.50 ^{b)} | 95 | 96 |
| 3-9 | 2-furyl | 0.25 | 83 | 96 |
| 3-10 | 2-thienyl | 0.50 | 94 | 92 |

| | | | | |
|---|---|---|---|---|
| a) The reaction temperature was 50°C b) The solvent was water: (CF₃)₂CHOH=30:1 | | | | |

### Example 4

The following reaction was carried out using iridium aqua complex (1-a-R).

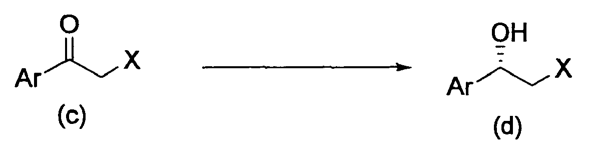

Iridium aqua complex (1-a-R) shown in Table 3 (the amount shown in Table 3 (mol%) relative to compound (c)), compound (c) (0.5 mmol) and 1.0 M formic acid aqueous solution (2.5 mL, 5 eq, adjusted with 4M sodium hydroxide in the case of pH=3.5, without adjustment in the case of pH=2.0) were mixed at room temperature, and the mixture was stirred. After confirming the disappearance of compound (c) by TLC, the mixture was extracted with dichloromethane, and the organic layer was dried over anhydrous Na₂SO₄, and concentrated. The residue was purified by silica gel column chromatography to give the corresponding compound (d). The conversion and ee were shown in Table 3.

**Table 3**

| | Ar | X | amount of use [mol%] | pH | conversion [%] | ee [%] |
|---|---|---|---|---|---|---|
| 4-1 | Ph- | NO₂ | 0.50 | 2.0 | 94 | 93 |
| 4-2 | 2-Me-Ph- | NO₂ | 0.50 | 2.0 | 93 | 80 |
| 4-3 | 4-^{t}Bu-Ph- | NO₂ | 0.50 | 2.0 | 92 | 99 |
| 4-4 | 3-Cl-Ph- | NO₂ | 0.50 | 2.0 | 95 | 95 |
| 4-5 | Ph- | Cl | 0.25 | 3.5 | 93 | 91 |
| 4-6 | Ph- | N₃ | 0.25 | 3.5 | 50 | 87 |

Example 5 Asymmetric transfer hydrogenation of nitroolefin compound

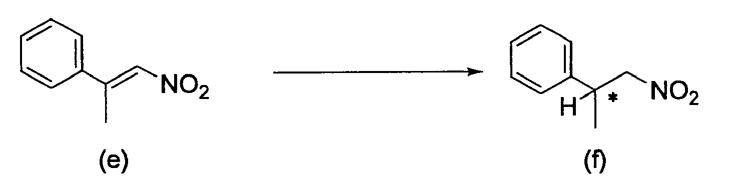

Iridium aqua complex shown in Table 4 (1 mol% relative to nitroolefin compound (e)), nitroolefin compound (e) (0.5 mmol) and 1.0 M formic acid aqueous solution (2.5 mL, 5 eq, adjusted with 4M sodium hydroxide in the case of pH=3.5, without adjustment in the case of pH=2.0) were mixed at room temperature, and the mixture was stirred. After confirming the disappearance of nitroolefin compound (e) by TLC, the mixture was extracted with dichloromethane, and the organic layer was dried over anhydrous Na₂SO₄, and concentrated. The residue was purified by silica gel column chromatography to give optically active nitroalkane compound (f). The conversion and ee were shown in Table 4. (S)-Nitroalkane compound (f) was obtained in Example 5-1 to 5-7, and (R)-nitroalkane compound (f) was obtained in Example 5-8.

**Table 4**

| | iridium aqua complex | pH | conversion [%] | ee [%] |
|---|---|---|---|---|
| 5-1 | (1-a-R) | 3.5 | 100 | 82 |
| 5-2 | (1-b-S) | 3.5 | 93 | 85 |
| 5-3 | (1-c-R) | 3.5 | 91 | 86 |
| 5-4 | (1-d-R) | 2.0 | 91 | 82 |
| 5-5 | (1-e-R) | 3.5 | 98 | 78 |
| 5-6 | (1-f-R) | 3.5 | 95 | 76 |
| 5-7 | (1-g-R) | 2.0 | 96 | 71 |
| 5-8 | (1-h-S) | 2.0 | 93 | 89 |

### Industrial Applicability

Since the chiral iridium aqua complex (1A) of the present invention has good solubility in water, the asymmetric transfer hydrogenation can be carried out in an aqueous solvent which is environmentally friendly and suitable for green chemistry. Even if the asymmetric transfer hydrogenation is carried out in the presence of formic acid or a salt thereof, it can be performed safely since the danger caused by hydrogen generation and the like is absent.
In addition, the chiral iridium aqua complex (1A) of the present invention has good stability to the air and water. Moreover, the asymmetric transfer hydrogenation using chiral iridium aqua complex (1A) of the present invention can achieve higher stereoselectivity and higher yield, as compared to conventional iridium complexes.

This application is based on patent application No. 2008-178040 filed in Japan, the contents of which are encompassed in full herein.

## Claims

1. A chiral iridium aqua complex represented by the formula (1A): 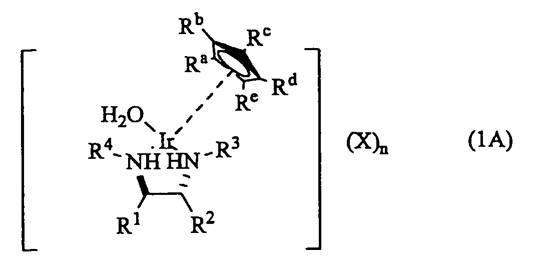 wherein
R^{a}, R^{b}, R^{c}, R^{d} and R^{e} are the same or different and each is a hydrogen atom, a methyl group, an ethyl group or a phenyl group,
R¹ and R² are the same or different and each is an aryl group optionally having substituent(s), or
R¹ and R² in combination show a C₃₋₄ straight chain alkylene group optionally having substituent(s) to form a ring,
R³ is an alkylsulfonyl group optionally having substituent(s) or an arylsulfonyl group optionally having substituent(s),
R⁴ is a hydrogen atom, an alkylsulfonyl group optionally having substituent(s) or an arylsulfonyl group optionally having substituent(s),
X is a monovalent or divalent anion, and
n is 2 when X is a monovalent anion, or n is 1 when X is a divalent anion.

2. A chiral iridium aqua complex represented by the formula (1): 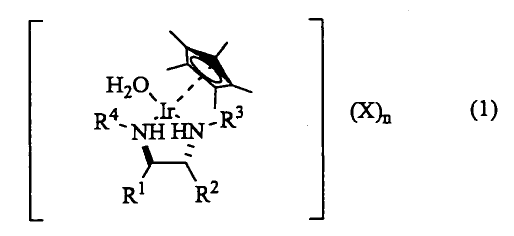 wherein
R¹ and R² are the same or different and each is an aryl group optionally having substituent(s), or
R¹ and R² in combination show a C₃₋₄ straight chain alkylene group optionally having substituent(s) to form a ring,
R³ is an alkylsulfonyl group optionally having substituent(s) or an arylsulfonyl group optionally having substituent(s),
R⁴ is a hydrogen atom, an alkylsulfonyl group optionally having substituent(s) or an arylsulfonyl group optionally having substituent(s),
X is a monovalent or divalent anion, and
n is 2 when X is a monovalent anion, or n is 1 when X is a divalent anion.

3. The chiral iridium aqua complex of claim 1 or 2, wherein
R¹ and R² are the same or different and each is a C₆₋₁₀ aryl group optionally having substituent(s) selected from a halogen atom, a C₁₋₆ alkyl group, a C₁₋₆ haloalkyl group, a C₁₋₆ alkoxy group and a C₁₋₆ haloalkoxy group; or
R¹ and R² in combination show a C₃₋₄ straight chain alkylene group optionally having substituent(s) to form a ring.

4. The chiral iridium aqua complex of claim 1 or 2, wherein R¹ and R² are the same or different and each is a C₆₋₁₀ aryl group optionally having substituent(s) selected from a halogen atom, a C₁₋₆ alkyl group, a C₁₋₆ haloalkyl group, a C₁₋₆ alkoxy group and a C₁₋₆ haloalkoxy group.

5. The chiral iridium aqua complex of claim 1 or 2, wherein R¹ and R² are the same or different and each is phenyl optionally having fluorine atom(s).

6. The chiral iridium aqua complex of claim 1 or 2, wherein R³ is a C₆₋₁₀ arylsulfonyl group optionally having substituent(s) selected from a halogen atom, a C₁₋₆ alkyl group, a C₁₋₆ haloalkyl group and a nitro group; or a C₁₋₆ alkylsulfonyl group optionally having halogen atom(s).

7. The chiral iridium aqua complex of claim 1 or 2, wherein R³ is phenylsulfonyl having substituent(s) selected from a fluorine atom, trifluoromethyl and nitro; or a C₁₋₄ alkylsulfonyl group having fluorine atom(s).

8. The chiral iridium aqua complex of claim 1 or 2, wherein R⁴ is a hydrogen atom; or a C₆₋₁₀ arylsulfonyl group optionally having C₁₋₆ alkyl group(s).

9. The chiral iridium aqua complex of claim 1 or 2, wherein R⁴ is a hydrogen atom.

10. The chiral iridium aqua complex of claim 1 or 2, wherein X is a sulfate ion.

11. A chiral iridium aqua complex represented by formula:

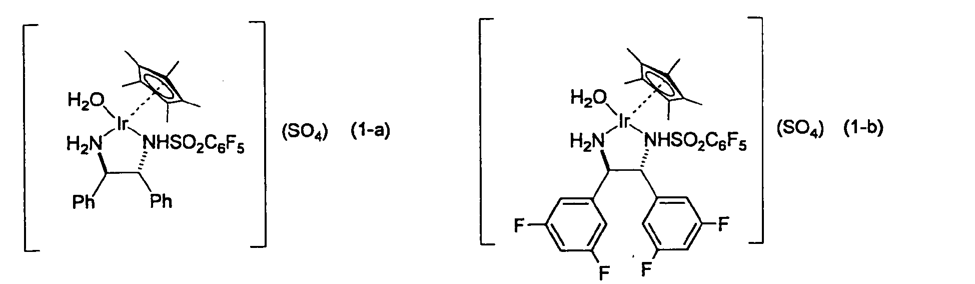

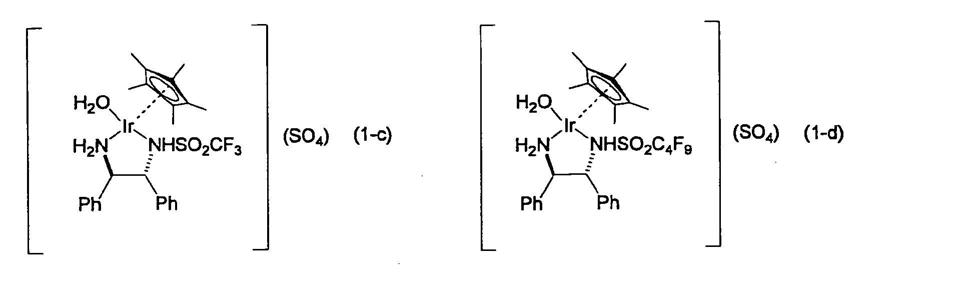

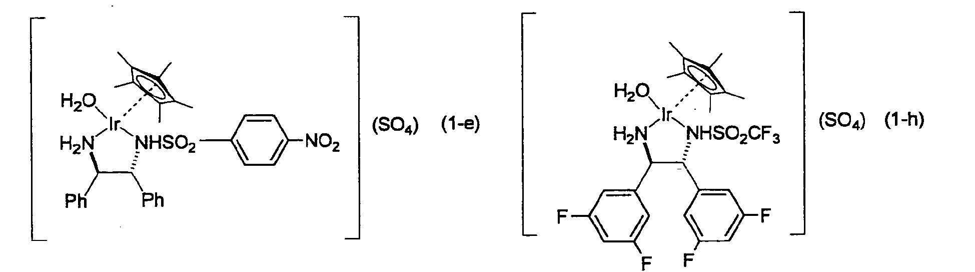

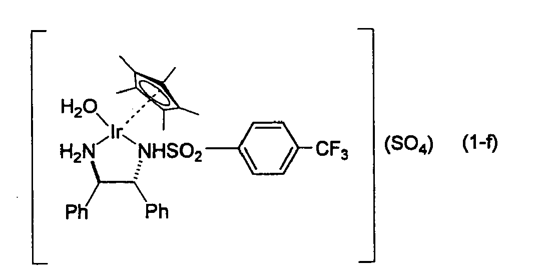

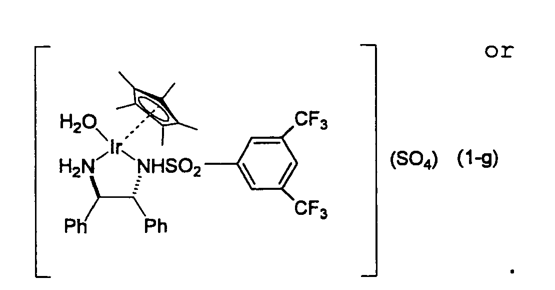

12. A chiral iridium aqua complex represented by formula: 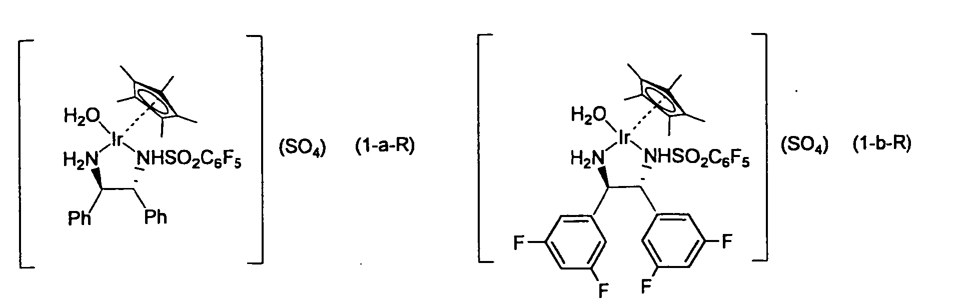 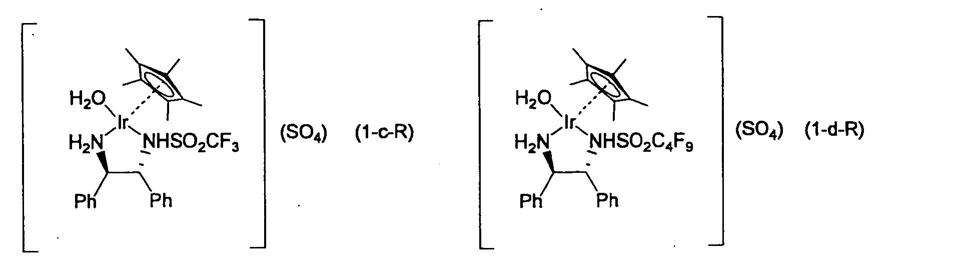 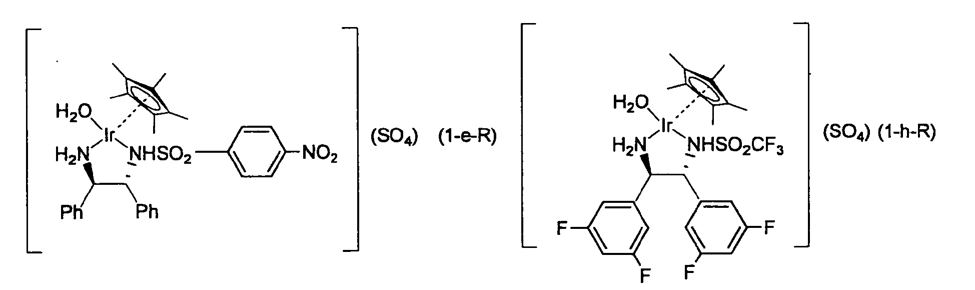 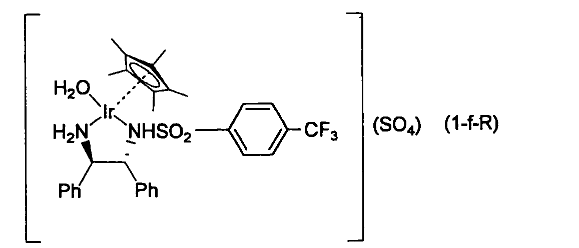 or

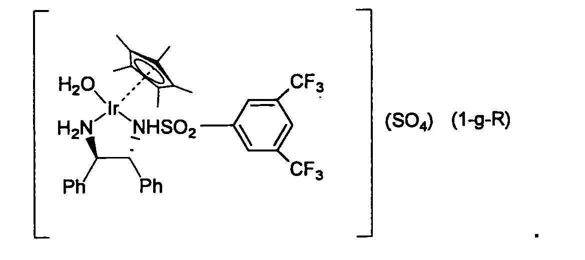

13. A chiral iridium aqua complex represented by formula: 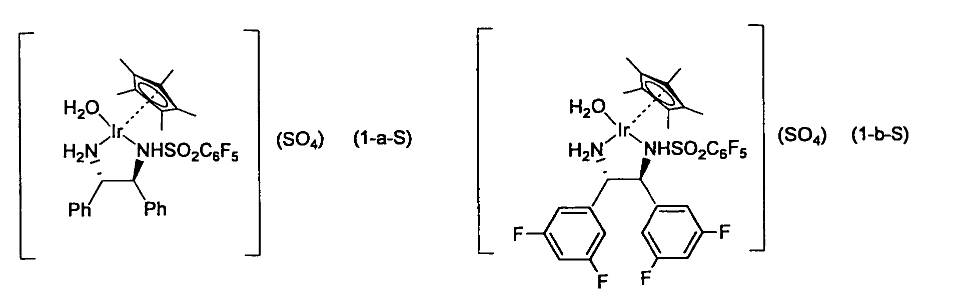 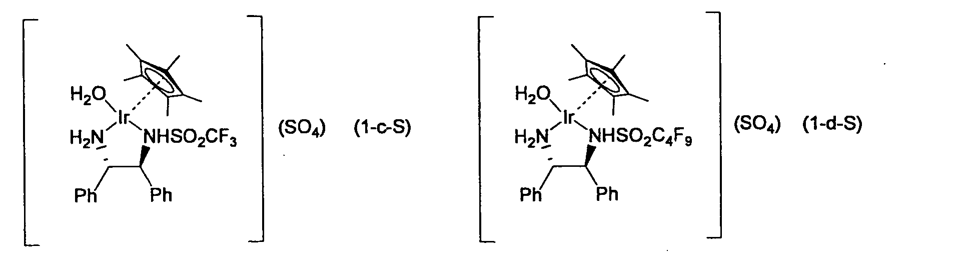 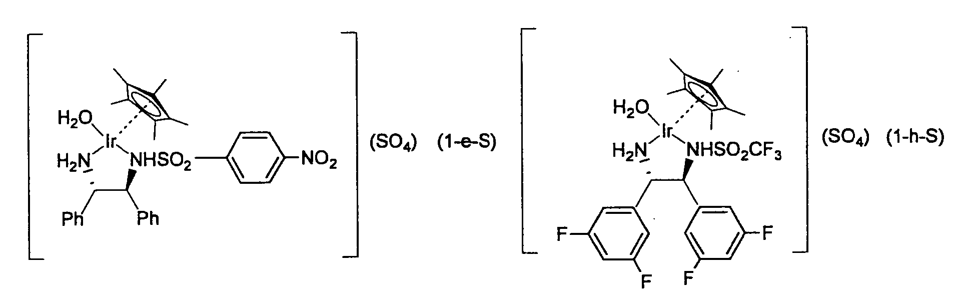 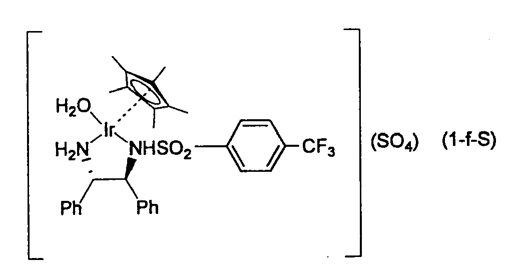 or

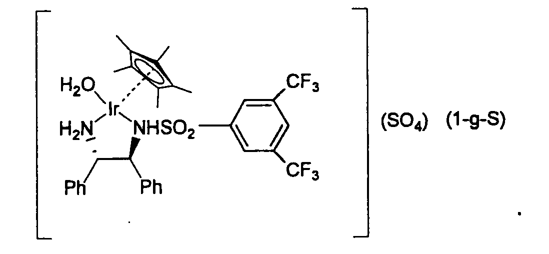

14. A chiral iridium aqua complex represented by formula: 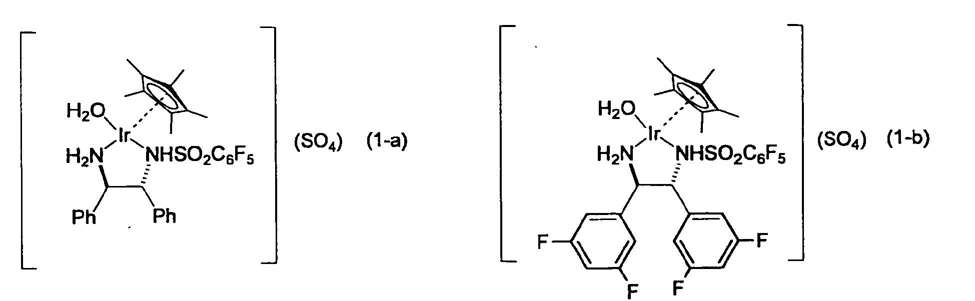 or

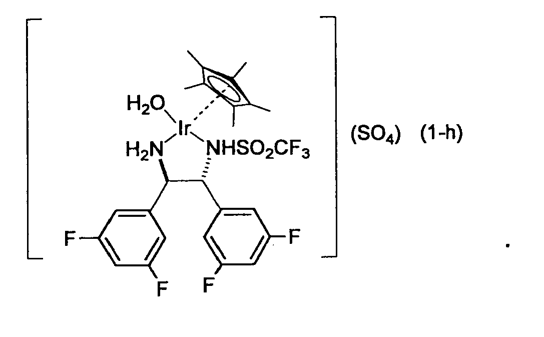

15. A chiral iridium aqua complex represented by formula: 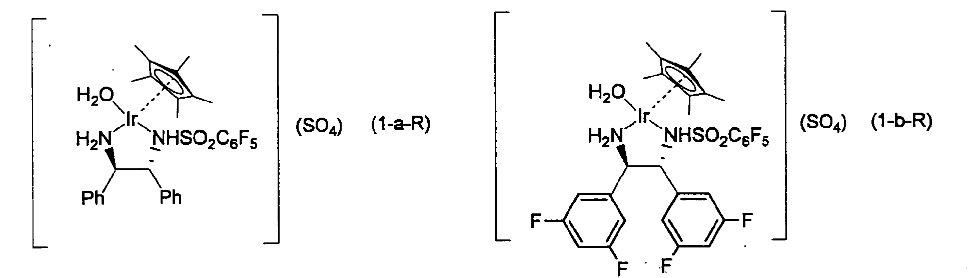 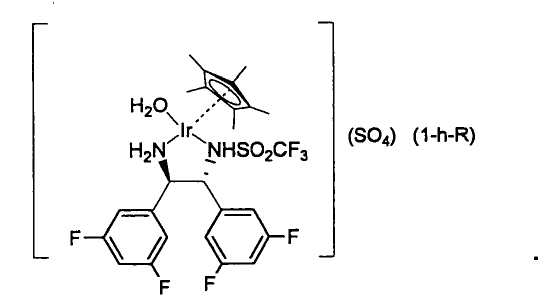 or

16. A chiral iridium aqua complex represented by formula: 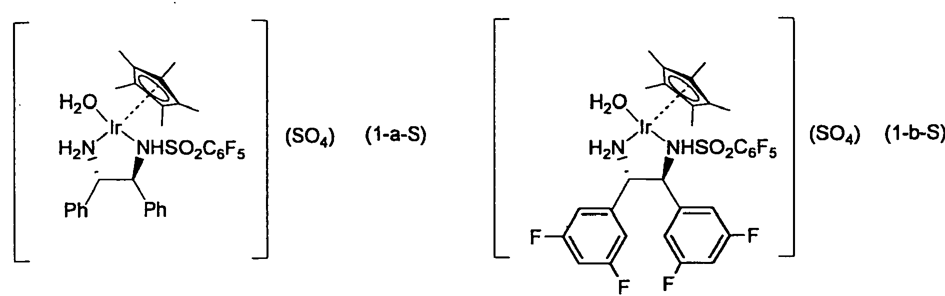 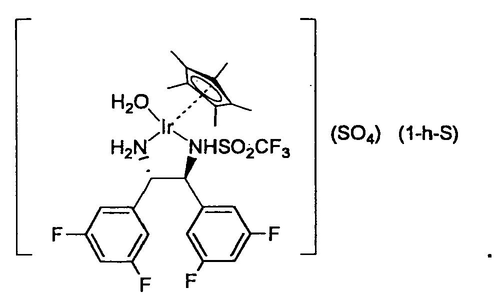 or

17. A method of producing a chiral iridium aqua complex represented by the formula (1A), which comprises reacting an iridium complex represented by the formula (2) with a chiral diamine represented by the formula (3): 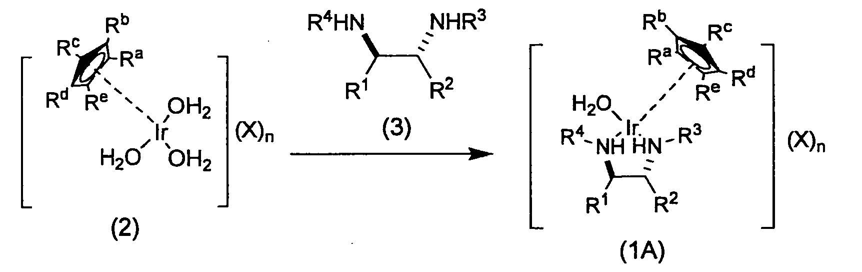 wherein
R^{a}, R^{b}, R^{c}, R^{d} and R^{e} are the same or different and each is a hydrogen atom, a methyl group, an ethyl group or a phenyl group,
R¹ and R² are the same or different and each is an aryl group optionally having substituent(s), or
R¹ and R² in combination show a C₃₋₄ straight chain alkylene group optionally having substituent(s) to form a ring,
R³ is an alkylsulfonyl group optionally having substituent(s) or an arylsulfonyl group optionally having substituent(s),
R⁴ is a hydrogen atom, an alkylsulfonyl group optionally having substituent(s) or an arylsulfonyl group optionally having substituent(s),
X is a monovalent or divalent anion, and
n is 2 when X is a monovalent anion, or n is 1 when X is a divalent anion.

18. A method of producing an optically active hydroxy compound represented by the formula (5), which comprises subjecting a carbonyl compound represented by the formula (4) to an asymmetric transfer hydrogenation in the presence of the chiral iridium aqua complex of any of claims 1 to 16: 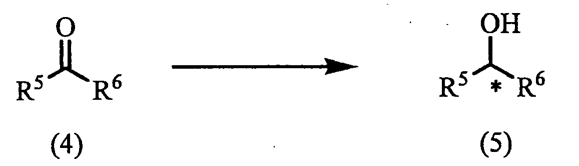 wherein
R⁵ is an aryl group optionally having substituent(s), a heteroaryl group optionally having substituent(s), a cycloalkyl group optionally having substituent(s) or an aralkyl group optionally having substituent(s),
R⁶ is a carboxyl group, a carbamoyl group optionally having substituent(s) or an alkyl group optionally having substituent(s), and
the carbon atom marked with * is an asymmetric carbon atom.

19. The method of claim 18, wherein the chiral iridium aqua complex is

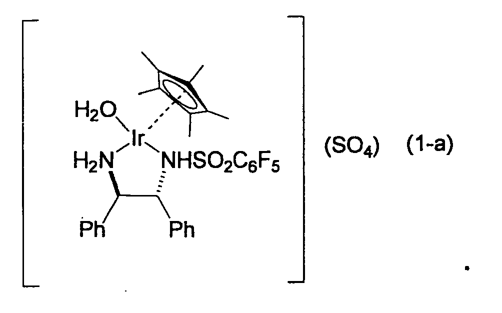

20. The method of claim 18, wherein the chiral iridium aqua complex is

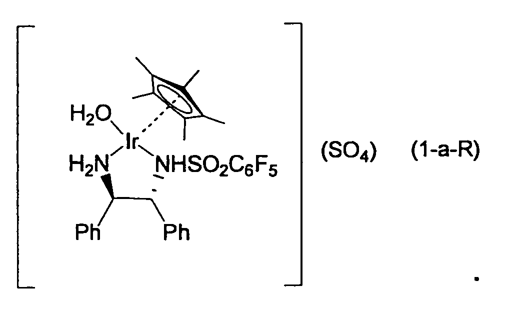

21. The method of claim 18, wherein the chiral iridium aqua complex is

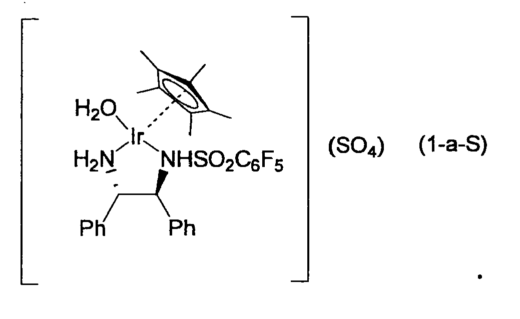

22. The method of claim 18, wherein R⁵ is a C₆₋₁₀ aryl group optionally having substituent(s) selected from a halogen atom, a C₁₋₆ alkyl group, a C₁₋₆ alkoxy group, a cyano group and nitro group; a C₃₋₈ cycloalkyl group; or a 5- or 6-membered heteroaryl group optionally having C₁₋₆ alkyl group(s).

23. The method of claim 18, wherein R⁶ is a C₁₋₆ alkyl group optionally having substituent(s) selected from a halogen atom, a cyano group, a nitro group and an azido group.

24. The method of claim 18, wherein the asymmetric transfer hydrogenation is carried out in the presence of formic acid or a salt thereof.

25. The method of claim 18, wherein the asymmetric transfer hydrogenation is carried out in the presence of formic acid.

26. The method of claim 18, wherein the asymmetric transfer hydrogenation is carried out under the condition of pH 2 to 5.

27. A method of producing an optically active nitroalkane compound represented by the formula (7), which comprises subjecting a nitroolefin compound represented by the formula (6) to an asymmetric transfer hydrogenation in the presence of the chiral iridium aqua complex of any of claims 1 to 16: 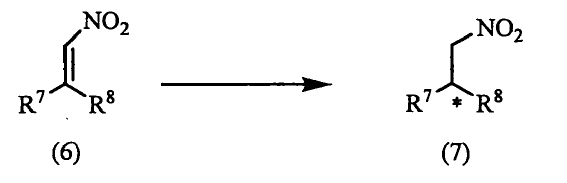 wherein
R⁷ is an aryl group optionally having substituent(s) or a heteroaryl group optionally having substituent(s),
R⁸ is an alkyl group optionally having substituent(s), an aryl group optionally having substituent(s) or a heteroaryl group optionally having substituent(s), and
the carbon atom marked with * is an asymmetric carbon atom.

28. The method of claim 27, wherein the chiral iridium aqua complex is

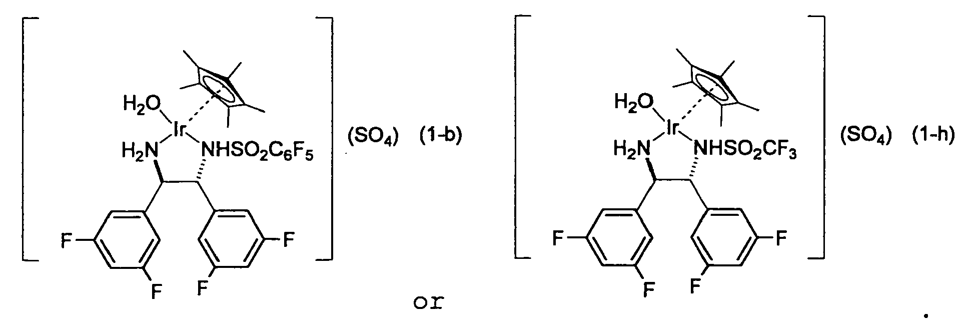

29. The method of claim 27, wherein the chiral iridium aqua complex is

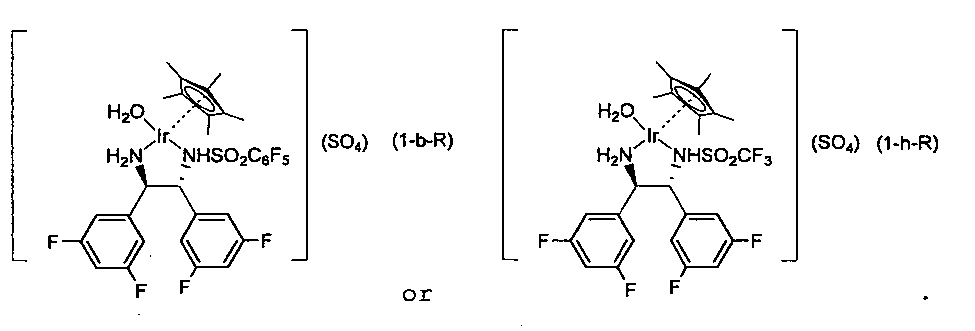

30. The method of claim 27, wherein the chiral iridium aqua complex is

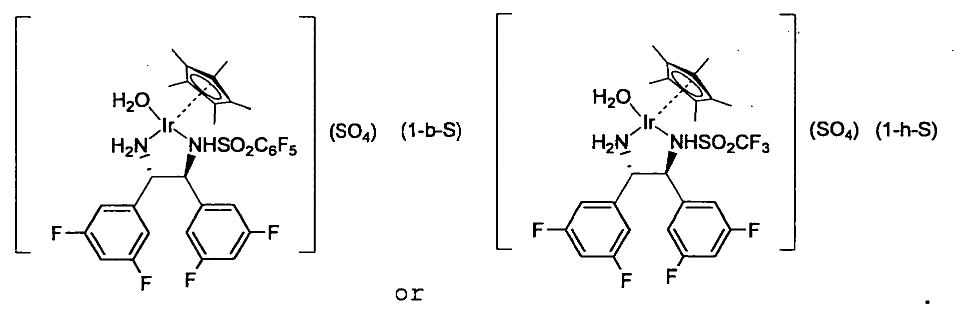

31. The method of claim 27, wherein R⁷ is a C₆₋₁₀ aryl group optionally having substituent(s) selected from a halogen atom, a C₁₋₆ alkyl group, a C₁₋₆ haloalkyl group and a C₁₋₆ alkoxy group.

32. The method of claim 27, wherein R⁸ is a C₁₋₆ alkyl group.

33. The method of claim 27, wherein the asymmetric transfer hydrogenation is carried out in the presence of formic acid or a salt thereof.

34. The method of claim 27, wherein the asymmetric transfer hydrogenation is carried out in the presence of formic acid.

35. The method of claim 27, wherein the asymmetric transfer hydrogenation is carried out under the condition of pH 2 to 5.

36. A chiral diamine represented by formula:

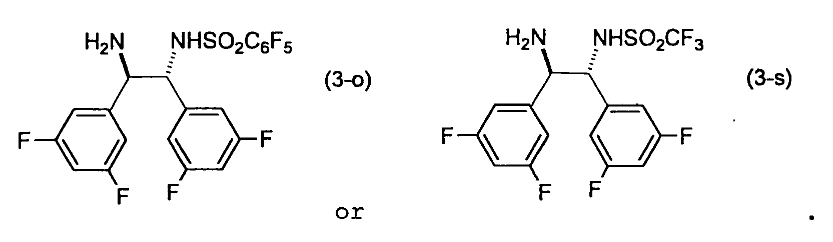

37. A chiral diamine represented by formula:

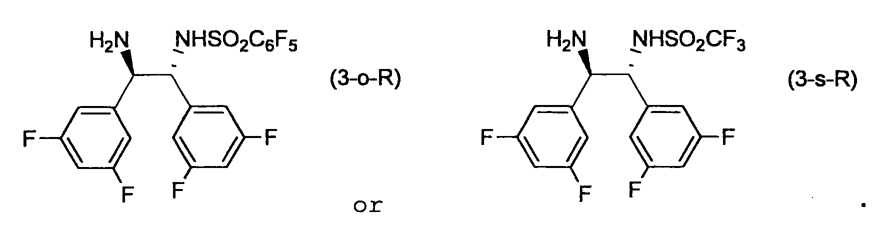

38. A chiral diamine represented by formula:
